# EUROPEAN PATENT SPECIFICATION

(11) **EP 2 501 300 B1**
(45) Date of publication and mention of the grant of the patent: **11.07.2018**
(21) Application number: 10830923.8
(22) Date of filing: 16.11.2010
(51) Int. Cl.: A61B 17/04, D02G 3/36, A61L 17/04, A61B 17/06, D04C 1/12, A61B 17/00

(54) **BRAIDED SELF-RETAINING SUTURES AND METHODS**
SELBSTHALTENDE GEFLOCHTENE NAHTEN UND VERFAHREN DAFÜR
SUTURES AUTORÉTENTIVES TRESSÉES ET PROCÉDÉS

(30) Priority: 16.11.2009 US 261660 P
(43) Date of publication of application: 26.09.2012
(73) Proprietor: Ethicon LLC, Guaynabo 00969 (PR)
(72) Inventor: AVELAR, Rui, Vancouver British Columbia V6J 2W7 (CA); D'AGOSTINO, William, L., Hamden Connecticut 06518 (US)
(74) Representative: Small, Gary James
(86) International application number: PCT/US2010/056898
(87) International publication number: WO 2011/060446

(56) References cited:
- WO-A2-2009/132284
- FR-A1- 2 926 452
- US-A1- 2006 155 328
- US-A1- 2007 005 110
- US-A1- 2008 082 113
- US-A1- 2008 312 688
- US-A1- 2009 012 560
- US-A1- 2009 112 259

## Description

### FIELD OF INVENTION

The present invention relates generally to self-retaining systems for surgical procedures, methods of manufacturing self-retaining systems for surgical procedures, and uses thereof.

### BACKGROUND OF INVENTION

Wound closure devices such as sutures, staples and tacks have been widely used in superficial and deep surgical procedures in humans and animals for closing wounds, repairing traumatic injuries or defects, joining tissues together (bringing severed tissues into approximation, closing an anatomical space, affixing single or multiple tissue layers together, creating an anastomosis between two hollow/luminal structures, adjoining tissues, attaching or reattaching tissues to their proper anatomical location), attaching foreign elements to tissues (affixing medical implants, devices, prostheses and other functional or supportive devices), and for repositioning tissues to new anatomical locations (repairs, tissue elevations, tissue grafting and related procedures) to name but a few examples.

Sutures are often used as wound closure devices. Sutures typically consist of a filamentous suture fiber or thread attached to a needle with a sharp point. Suture fibers or threads can be made from a wide variety of materials including bioabsorbable (i.e., that break down completely in the body over time), or non-absorbable (permanent; non-degradable) materials. Absorbable sutures have been found to be particularly useful in situations where suture removal might jeopardize the repair or where the natural healing process renders the support provided by the suture material unnecessary after wound healing has been completed; as in, for example, completing an uncomplicated skin closure. Non-degradable (non-absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomosis. Also, a wide variety of surgical needles are available, and the shape, and size of the needle body and the configuration of the needle tip is typically selected based upon the needs of the particular application.

To use an ordinary suture, the suture needle is advanced through the desired tissue on one side of the wound and then through the adjacent side of the wound. The suture is then formed into a "loop" which is completed by tying a knot in the suture to hold the wound closed. Knot tying takes time and causes a range of complications, including, but not limited to (i) spitting (a condition where the suture, usually a knot) pushes through the skin after a subcutaneous closure), (ii) infection (bacteria are often able to attach and grow in the spaces created by a knot), (iii) bulk/mass (a significant amount of suture material left in a wound is the portion that comprises the knot), (iv) slippage (knots can slip or come untied), and (v) irritation (knots serve as a bulk "foreign body" in a wound). Suture loops associated with knot tying may lead to ischemia (knots can create tension points that can strangulate tissue and limit blood flow to the region) and increased risk of dehiscence or rupture at the surgical wound. Knot tying is also labor intensive and can comprise a significant percentage of the time spent closing a surgical wound. Additional operative procedure time is not only bad for the patient (complication rates rise with time spent under anesthesia), but it also adds to the overall cost of the operation (many surgical procedures are estimated to cost between $15 and $30 per minute of operating time).

Self-retaining sutures (including barbed sutures) differ from conventional sutures in that self-retaining sutures possess numerous tissue retainers (such as barbs) which anchor the self-retaining suture into the tissue following deployment and resist movement of the suture in a direction opposite to that in which the retainers face, thereby eliminating the need to tie knots to affix adjacent tissues together (a "knotless" closure). Knotless tissue-approximating devices having barbs have been previously described in, for example, U.S. Pat. No. 5,374,268, disclosing armed anchors having barb-like projections, while suture assemblies having barbed lateral members have been described in U.S. Pat. Nos. 5,584,859 and 6,264,675. Sutures having a plurality of barbs positioned along a greater portion of the suture are described in U.S. Pat No. 5,931,855, which discloses a unidirectional barbed suture, and U.S. Pat. No. 6,241,747, which discloses a bidirectional barbed suture. Methods and apparatus for forming barbs on sutures have been described in, for example, U.S. Pat. Nos. 6,848,152. Self-retaining systems for wound closure also result in better approximation of the wound edges, evenly distribute the tension along the length of the wound (reducing areas of tension that can break or lead to ischemia), decrease the bulk of suture material remaining in the wound (by eliminating knots) and reduce spitting (the extension of suture material - typically knots - through the surface of the skin. All of these features are thought to reduce scarring, improve cosmesis, and increase wound strength relative to wound closures using plain sutures or staples. Thus, self-retaining sutures, because such sutures avoid knot tying, allow patients to experience an improved clinical outcome, and also save time and costs associated with extended surgeries and follow-up treatments.

The ability of self-retaining sutures to anchor and hold tissues in place even in the absence of tension applied to the suture by a knot is a feature that also provides superiority over plain sutures. When closing a wound that is under tension, this advantage manifests itself in several ways: (i) self-retaining sutures have a multiplicity of retainers which can dissipate tension along the entire length of the suture (providing hundreds of "anchor" points this produces a superior cosmetic result and lessens the chance that the suture will "slip" or pull through) as opposed to knotted interrupted sutures which concentrate the tension at discrete points; (ii) complicated wound geometries can be closed (circles, arcs, jagged edges) in a uniform manner with more precision and accuracy than can be achieved with interrupted sutures; (iii) self-retaining sutures eliminate the need for a "third hand" which is often required for maintaining tension across the wound during traditional suturing and knot tying (to prevent "slippage" when tension is momentarily released during tying); (iv) self-retaining sutures are superior in procedures where knot tying is technically difficult, such as in deep wounds or laparoscopic/endoscopic procedures; and (v) self-retaining sutures can be used to approximate and hold the wound prior to definitive closure. As a result, self-retaining sutures provide easier handling in anatomically tight or deep places (such as the pelvis, abdomen and thorax) and make it easier to approximate tissues in laparoscopic/endoscopic and minimally invasive procedures; all without having to secure the closure via a knot. Greater accuracy allows self-retaining sutures to be used for more complex closures (such as those with diameter mismatches, larger defects or purse string suturing) than can be accomplished with plain sutures.

A self-retaining suture may be unidirectional, having one or more retainers oriented in one direction along the length of the suture fiber or thread; or bidirectional, typically having one or more retainers oriented in one direction along a portion of the fiber or thread, followed by one or more retainers oriented in another (often opposite) direction over a different portion of the fiber or thread (as described with barbed retainers in U.S. Pat. Nos. 5,931,855 and. 6,241,747). Although any number of sequential or intermittent configurations of retainers are possible, a common form of bidirectional self-retaining suture involves a needle at one end of a suture fiber or thread which has barbs having tips projecting "away" from the needle until the transition point (often the midpoint) of the suture is reached; at the transition point the configuration of barbs reverses itself about 180° (such that the barbs are now facing in the opposite direction) along the remaining length of the suture fiber or thread before attaching to a second needle at the opposite end (with the result that the barbs on this portion of the suture also have tips projecting "away" from the nearest needle). Projecting "away" from the needle means that the tip of the barb is further away from the needle and the portion of suture comprising the barb may be pulled more easily through tissue in the direction of the needle than in the opposite direction. Put another way, the barbs on both "halves" of a typical bidirectional self-retaining suture have tips that point towards the middle, with a transition segment (lacking barbs) interspersed between them, and with a needle attached to either end.

For surgical applications involving engaging soft tissue with bone, and approximating soft, but tough tissue, such as fascia, capsule, meniscus-like structures, glenoid labrum in the shoulder, flexor tendons, tendons, rotator cuffs, and the like, braided suture has been used. Such uses require suture materials that are strong in order to hold the tissue together. Braided sutures are flexible and are also strong, having good tensile strength and can successfully hold such tissue together.

WO 2009/132284 discloses a self-retaining suture comprising a filament which has retainers on the surface. The filament may be a plurality of filaments braided together and the suture may have a triangular or circular cross-section.

US 2006/155328 discloses a surgical suture having a core formed of a plurality of twisted fibres of a first material, surrounded by a braided cover made from fibres of a second material different from the first.

FR 2 926 452 discloses a surgical thread with hooking pins for tissues and an inner elongation limiter. The inner elongation limiter may be formed of a helically wound filament, or by successive links of a chain, or by multiple links of a chain.

### SUMMARY OF INVENTION

Despite the multitude of advantages of unidirectional and bidirectional self-retaining sutures, there remains a need to improve upon the design of the suture such that a variety of common limitations can be eliminated. Specifically, several problems common to existing self-retaining sutures can be addressed by the embodiments of this disclosure, including, but not limited to: (i) retainers or barbs that are fragile and break or are too flexible and bend back, or do not stand proud due to an insufficient ability of the material to plastically deform and as such do not properly engage when deployed in tissue; (ii) inadequate "hold" provided by the retainers for some surgical procedures; resulting in retainers or barbs do not sufficiently anchor in the surrounding tissue and "pull through;" (iii) insufficient contact between the retainers and the surrounding tissue (often occurring when the fiber or thread diameter is too small relative to the diameter of the hole created by a larger needle; this limits the ability of the retainers to contact and "grip" the surrounding tissue); (iv) breakage of the self-retaining suture during tensioning and wound approximation; and (v) rotation and slippage of the retainers after deployment. Furthermore, the creation and/ or deployment of retainer features of self-retaining sutures may be difficult to achieve.

Thus, it would be desirable to provide improved self-retaining sutures which have enhanced ability to anchor into the surrounding tissue, enhanced tissue holding capabilities, enhanced maximum load, and enhanced clinical performance.

It would further be desirable to provide improved methods for making self-retaining sutures that yield retainers which can be more readily created, elevated and deployed.

In accordance with the foregoing background and the limitations of the prior art, the present disclosure provides, improved self-retaining sutures which have enhanced ability to anchor into the surrounding tissue, enhanced tissue holding capabilities, enhanced maximum load, and enhanced clinical performance and methods for making such self-retaining sutures.

The invention is defined by the appended claims.

The details of one or more embodiments are set forth in the description below. Other features, objects and advantages will be apparent from the description, the drawings, and the claims.

### BRIEF DESCRIPTION OF THE DRAWINGS

Features of the invention, its nature and various advantages will be apparent from the accompanying drawings and the following detailed description of various embodiments.
FIGS. 1A and 1B are perspective views of a self-retaining suture comprising a braided core in accordance with an embodiment of the present invention.
FIG. 1C is a sectional view of the suture of FIGS. 1A and 1B illustrating the arrangement of the braided core and sheath.
FIG. 1D is a sectional view of an alternative self-retaining braided suture.
FIG. 1E is a partial sectional view of a self-retaining braided ribbon in accordance with an embodiment of the present invention.
FIG. 1F is a partial sectional view of a self-retaining braided ring in accordance with an embodiment of the present invention.
FIGS. 2A, 2B and 2C are perspective views illustrating steps in the creation of a retainer of a self-retaining braided suture comprising braided core and sheath in accordance with an embodiment of the present invention.
FIG. 2D is a perspective view illustrating an alternative method to create a retainer of a self-retaining braided suture comprising braided core and sheath in accordance with an embodiment of the present invention.
FIG. 2E is a perspective view illustrating an alternative method to create a retainer of a self-retaining braided suture comprising braided core and sheath in accordance with an embodiment of the present invention.
FIGS. 3A, 3B and 3C are perspective views illustrating steps in the creation of a retainer of a self-retaining braided suture in accordance with an embodiment of the present disclosure.
FIGS. 3D and 3E are schematic views illustrating an alternative method to create a self-retaining braided suture in accordance with an embodiment of the present disclosure.
FIG. 3F is a schematic view illustrating an alternative method to create a self-retaining braided suture in accordance with an embodiment of the present disclosure.
FIGS. 3G is a perspective view illustrating another alternative method to create a self-retaining braided suture in accordance with an embodiment of the present disclosure.
FIG. 4A is a perspective view of a retainer that may be added to a braided suture fiber or thread to form a self-retaining braided suture in accordance with an embodiment of the present disclosure.
FIG. 4B is a perspective view of a self-retaining braided suture including a plurality of the retainers of FIG. 4A in accordance with an embodiment of the present disclosure.
FIG. 4C is a perspective view showing deployment of the retainers of the self-retaining braided suture of FIG. 4B.
FIG. 5A illustrates a method and apparatus for extruding a material over a braided core to form a braided suture suitable for creation of a self-retaining braided suture in accordance with an embodiment of the present invention.

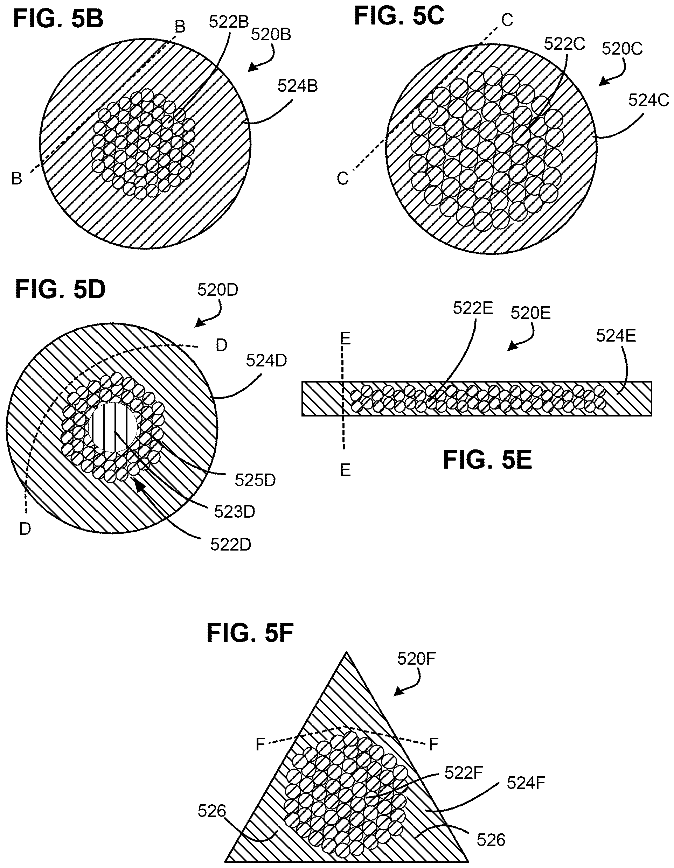

FIGS. 5B, 5C, 5D, 5F illustrate alternative configurations of extruded suture stock suitable for creation of a self-retaining suture comprising a sheath over a braided core in accordance with embodiments of the present disclosure. FIG. 5E illustrates an alternative configuration of extruded suture stock suitable for creation of a self-retaining suture comprising a sheath over a braided core in accordance with embodiments of the present invention. In these embodiments (FIGS. 5B-5F), the braided core has good tensile strength and can also be elastic and flexible. The retainers formed have a large plastic deformation zone and can, for example, be work hardened in order to form retainers that are, for example, stiff.

### DETAILED DESCRIPTION

### DEFINITIONS

Definitions of certain terms that may be used hereinafter include the following.

"Self-retaining system" refers to a self-retaining suture together with devices for deploying the suture into tissue. Such deployment devices include, without limitation, suture needles and other deployment devices as well as sufficiently rigid and sharp ends on the suture itself to penetrate tissue.

"Self-retaining suture" refers to a suture that comprises features on the suture for engaging tissue without the need for a knot or suture anchor.

"Tissue retainer" (or simply "retainer") or "barb" refers to a physical feature of a suture which is adapted to mechanically engage tissue and resist movement of the suture in at least one axial directions. By way of example only, tissue retainer or retainers can include hooks, projections, barbs, darts, extensions, bulges, anchors, protuberances, spurs, bumps, points, cogs, tissue engagers, traction devices, surface roughness, surface irregularities, surface defects, edges, facets and the like. In certain configurations, tissue retainers are adapted to engage tissue to resist movement of the suture in a direction other than the direction in which the suture is deployed into the tissue by the surgeon, by being oriented to substantially face the deployment direction. In some embodiments the retainers lie flat when pulled in the deployment direction and open or "fan out" when pulled in a direction contrary to the deployment direction. As the tissue-penetrating end of each retainer faces away from the deployment direction when moving through tissue during deployment, the tissue retainers should not catch or grab tissue during this phase. Once the self-retaining suture has been deployed, a force exerted in another direction (often substantially opposite to the deployment direction) causes the retainers to be displaced from the deployment position (i.e. resting substantially along the suture body), forces the retainer ends to open (or "fan out") from the suture body in a manner that catches and penetrates into the surrounding tissue, and results in tissue being caught between the retainer and the suture body; thereby "anchoring" or affixing the self-retaining suture in place. In certain other embodiments, the tissue retainers may be configured to permit motion of the suture in one direction and resist movement of the suture in another direction without fanning out or deploying. In certain other configurations, the tissue retainer may be configured or combined with other tissue retainers to resist motion of the suture in both directions. Typically a suture having such retainers is deployed through a device such as a cannula which prevents contact between the retainers and the tissue until the suture is in the desired location.

"Retainer configurations" refers to configurations of tissue retainers and can include features such as size, shape, flexibility, surface characteristics, and so forth. These are sometimes also referred to as "barb configurations".

"Bidirectional suture" refers to a self-retaining suture having retainers oriented in one direction at one end and retainers oriented in the other direction at the other end. A bidirectional suture is typically armed with a needle at each end of the suture thread. Many bidirectional sutures have a transition segment located between the two barb orientations.

"Transition segment" refers to a retainer-free (barb-free) portion of a bidirectional suture located between a first set of retainers (barbs) oriented in one direction and a second set of retainers (barbs) oriented in another direction. The transition segment can be at about the midpoint of the self-retaining suture, or closer to one end of the self-retaining suture to form an asymmetrical self-retaining suture system.

"Suture fiber or thread" refers to the filamentary body component of the suture. The suture fiber or thread may be a monofilament, or comprise multiple fibers or filaments or threads as in a braided suture. The suture thread may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

"Monofilament suture" refers to a suture comprising a monofilamentary suture thread.

"Braided suture" refers to a suture comprising a multifilamentary suture thread. The plurality of filaments or fibers or threads in such suture fibers or threads are typically braided, twisted, or woven together. As used herein, "braided suture" may also refer to a suture having a braided core and a non-braided sheath or coating.

"Degradable suture" (also referred to as "biodegradable suture" or "absorbable suture") refers to a suture which, after introduction into a tissue is broken down and absorbed by the body. Typically, the degradation process is at least partially mediated by, or performed in, a biological system. "Degradation" refers to a chain scission process by which a polymer chain is cleaved into oligomers and monomers. Chain scission may occur through various mechanisms, including, for example, by chemical reaction (e.g., hydrolysis, oxidation/reduction, enzymatic mechanisms or a combination of these) or by a thermal or photolytic process. Polymer degradation may be characterized, for example, using gel permeation chromatography (GPC), which monitors the polymer molecular mass changes during erosion and breakdown. Degradable suture material may include polymers such as polyglycolic acid, copolymers of glycolide and lactide, copolymers of trimethylene carbonate and glycolide with diethylene glycol (e.g., MAXONTM, Tyco Healthcare Group), terpolymer composed of glycolide, trimethylene carbonate, and dioxanone (e.g., BIOSYNTM [glycolide (60%), trimethylene carbonate (26%), and dioxanone (14%)], Tyco Healthcare Group), copolymers of glycolide, caprolactone, trimethylene carbonate, and lactide (e.g., CAPROSYNTM, Tyco Healthcare Group). A dissolvable suture can also include partially deacetylated polyvinyl alcohol. Polymers suitable for use in degradable sutures can be linear polymers, branched polymers or multi-axial polymers. Examples of multi-axial polymers used in sutures are described in U.S. Patent Application Publication Nos. 20020161168, 20040024169, and 20040116620. Sutures made from degradable suture material lose tensile strength as the material degrades. Degradable sutures can be in either a braided multifilament form or a monofilament form.

"Non-degradable suture" (also referred to as "non-absorbable suture") refers to a suture comprising material that is not degraded by chain scission such as chemical reaction processes (e.g., hydrolysis, oxidation/reduction, enzymatic mechanisms or a combination of these) or by a thermal or photolytic process. Non-degradable suture material includes polyamide (also known as nylon, such as nylon 6 and nylon 6,6), polyester (e.g., polyethylene terephthlate), polytetrafluoroethylene (e.g., expanded polytetrafluoroethylene), polyether-ester such as polybutester (block copolymer of butylene terephthalate and polytetra methylene ether glycol), polyurethane, metal alloys, metal (e.g., stainless steel wire), polypropylene, polyethelene, silk, and cotton. Sutures made of non-degradable suture material are suitable for applications in which the suture is meant to remain permanently or is meant to be physically removed from the body.

"Suture diameter" refers to the diameter of the body of the suture. It is to be understood that a variety of suture lengths may be used with the sutures described herein and that while the term "diameter" is often associated with a circular periphery, it is to be understood herein to indicate a cross-sectional dimension associated with a periphery of any shape. Suture sizing is based upon diameter. United States Pharmacopeia ("USP") designation of suture size runs from 0 to 7 in the larger range and 1-0 to 11-0 in the smaller range; in the smaller range, the higher the value preceding the hyphenated zero, the smaller the suture diameter. The actual diameter of a suture will depend on the suture material, so that, by way of example, a suture of size 5-0 and made of collagen will have a diameter of 0.15 mm, while sutures having the same USP size designation but made of a synthetic absorbable material or a non-absorbable material will each have a diameter of 0.1 mm. The selection of suture size for a particular purpose depends upon factors such as the nature of the tissue to be sutured and the importance of cosmetic concerns; while smaller sutures may be more easily manipulated through tight surgical sites and are associated with less scarring, the tensile strength of a suture manufactured from a given material tends to decrease with decreasing size. It is to be understood that the sutures and methods of manufacturing sutures disclosed herein are suited to a variety of diameters, including without limitation 7, 6, 5, 4, 3, 2, 1, 0, 1-0, 2-0, 3-0, 4-0, 5-0, 6-0, 7-0, 8-0, 9-0, 10-0 and 11-0.

"Suture deployment end" refers to an end of the suture to be deployed into tissue; one or both ends of the suture may be suture deployment ends. The suture deployment end may be attached to a deployment device such as a suture needle, or may be sufficiently sharp and rigid to penetrate tissue on its own.

"Armed suture" refers to a suture having a suture needle on at least one suture deployment end.

"Needle attachment" refers to the attachment of a needle to a suture requiring same for deployment into tissue, and can include methods such as crimping, swaging, using adhesives, and so forth. The suture fiber or thread is attached to the suture needle using methods such as crimping, swaging and adhesives. Attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. US 2004/0088003). The point of attachment of the suture to the needle is known as the swage.

"Suture needle" refers to needles used to deploy sutures into tissue, which come in many different shapes, forms and compositions. There are two main types of needles, traumatic needles and atraumatic needles. Traumatic needles have channels or drilled ends (that is, holes or eyes) and are supplied separate from the suture fiber or thread and are threaded on site. Atraumatic needles are eyeless and are attached to the suture at the factory by swaging or other methods whereby the suture material is inserted into a channel at the blunt end of the needle which is then deformed to a final shape to hold the suture and needle together. As such, atraumatic needles do not require extra time on site for threading and the suture end at the needle attachment site is generally smaller than the needle body. In the traumatic needle, the fiber or thread comes out of the needle's hole on both sides and often the suture rips the tissues to a certain extent as it passes through. Most modern sutures are swaged atraumatic needles. Atraumatic needles may be permanently swaged to the suture or may be designed to come off the suture with a sharp straight tug. These "pop-offs" are commonly used for interrupted sutures, where each suture is only passed once and then tied. For barbed sutures that are uninterrupted, these atraumatic needles are preferred.

Suture needles may also be classified according to the geometry of the tip or point of the needle. For example, needles may be (i) "tapered" whereby the needle body is round and tapers smoothly to a point; (ii) "cutting" whereby the needle body is triangular and has a sharpened cutting edge on the inside; (iii) "reverse cutting" whereby the cutting edge is on the outside; (iv) "trocar point" or "taper cut" whereby the needle body is round and tapered, but ends in a small triangular cutting point; (v) "blunt" points for sewing friable tissues; (vi) "side cutting" or "spatula points" whereby the needle is flat on top and bottom with a cutting edge along the front to one side (these are typically used for eye surgery).

Suture needles may also be of several shapes including, (i) straight, (ii) half curved or ski, (iii) 1/4 circle, (iv) 3/8 circle, (v) 1/2 circle, (vi) 5/8 circle, (v) and compound curve.

Suturing needles are described, for example, in US Patent Nos. 6,322,581 and 6,214,030 (Mani, Inc., Japan); and 5,464,422 (W.L. Gore, Newark, DE); and 5,941,899; 5,425,746; 5,306,288 and 5,156,615 (US Surgical Corp., Norwalk, CT); and 5,312,422 (Linvatec Corp., Largo, FL); and 7,063,716 (Tyco Healthcare, North Haven, CT). Other suturing needles are described, for example, in US Patent Nos. 6,129,741; 5,897,572; 5,676,675; and 5,693,072. The sutures described herein may be deployed with a variety of needle types (including without limitation curved, straight, long, short, micro, and so forth), needle cutting surfaces (including without limitation, cutting, tapered, and so forth), and needle attachment techniques (including without limitation, drilled end, crimped, and so forth). Moreover, the sutures described herein may themselves include sufficiently rigid and sharp ends so as to dispense with the requirement for deployment needles altogether.

"Needle diameter" refers to the diameter of a suture deployment needle at the widest point of that needle. While the term "diameter" is often associated with a circular periphery, it is to be understood herein to indicate a cross-sectional dimension associated with a periphery of any shape.

"Wound closure" refers to a surgical procedure for closing of a wound. An injury, especially one in which the skin or another external or internal surface is cut, torn, pierced, or otherwise broken is known as a wound. A wound commonly occurs when the integrity of any tissue is compromised (e.g., skin breaks or burns, muscle tears, or bone fractures). A wound may be caused by an act, such as a puncture, fall, or surgical procedure; by an infectious disease; or by an underlying medical condition. Surgical wound closure facilitates the biological event of healing by joining, or closely approximating, the edges of those wounds where the tissue has been torn, cut, or otherwise separated. Surgical wound closure directly apposes or approximates the tissue layers, which serves to minimize the volume new tissue formation required to bridge the gap between the two edges of the wound. Closure can serve both functional and aesthetic purposes. These purposes include elimination of dead space by approximating the subcutaneous tissues, minimization of scar formation by careful epidermal alignment, and avoidance of a depressed scar by precise eversion of skin edges.

'Tissue elevation procedure" refers to a surgical procedure for repositioning tissue from a lower elevation to a higher elevation (i.e. moving the tissue in a direction opposite to the direction of gravity). The retaining ligaments of the face support facial soft tissue in the normal anatomic position. However, with age, gravitational effects and loss of tissue volume effect downward migration of tissue, and fat descends into the plane between the superficial and deep facial fascia, thus causing facial tissue to sag. Face-lift procedures are designed to lift these sagging tissues, and are one example of a more general class of medical procedure known as a tissue elevation procedure. More generally, a tissue elevation procedure reverses the appearance change that results from effects of aging and gravity over time, and other temporal effects that cause tissue to sag, such as genetic effects. It should be noted that tissue can also be repositioned without elevation; in some procedures tissues are repositioned laterally (away from the midline), medially (towards the midline) or inferiorly (lowered) in order to restore symmetry (i.e. repositioned such that the left and right sides of the body "match").

"Medical device" or "implant" refers to any object placed in the body for the purpose of restoring physiological function, reducing/alleviating symptoms associated with disease, and/or repairing and/or replacing damaged or diseased organs and tissues. While normally composed of biologically compatible synthetic materials (e.g., medical-grade stainless steel, titanium and other metals or polymers such as polyurethane, silicon, PLA, PLGA and other materials) that are exogenous, some medical devices and implants include materials derived from animals (e.g., "xenografts" such as whole animal organs; animal tissues such as heart valves; naturally occurring or chemically-modified molecules such as collagen, hyaluronic acid, proteins, carbohydrates and others), human donors (e.g., "allografts" such as whole organs; tissues such as bone grafts, skin grafts and others), or from the patients themselves (e.g., "autografts" such as saphenous vein grafts, skin grafts, tendon/ligament/muscle transplants). Medical devices that can be used in procedures in conjunction with the present invention include, but are not restricted to, orthopedic implants (artificial joints, ligaments and tendons; screws, plates, and other implantable hardware), dental implants, intravascular implants (arterial and venous vascular bypass grafts, hemodialysis access grafts; both autologous and synthetic), skin grafts (autologous, synthetic), tubes, drains, implantable tissue bulking agents, pumps, shunts, sealants, surgical meshes (e.g., hernia repair meshes, tissue scaffolds), fistula treatments, spinal implants (e.g., artificial intervertebral discs, spinal fusion devices, etc.) and the like.

### SELF-RETAINING BRAIDED SUTURE SYSTEM

As discussed above, the present disclosure provides compositions, configurations, methods of manufacturing and methods of using self-retaining systems in surgical procedures which greatly increase the ability of the self-retaining sutures to anchor into the surrounding tissue to provide superior holding strength and improve clinical performance.

Self-retaining sutures have previously been made from monofilament suture threads. However, conventional braided sutures have useful properties in particular surgical applications. For example, braided sutures such as VICRYL™, SURGICRYL™, BIOVEK™, VISORB™, POLYSORB™, SURGISORB™, and DEXON™ are useful in applications where it is desirable to have an absorbable suture which retains its tensile strength for a period of three to four weeks and is completely absorbed by hydrolysis in a period of ten weeks. These sutures are manufactured using braided polyglycolic acid (PGA) material and are in some cases coated with polycaprolactone and calcium stearate. Braided sutures are also often used in arthroscopic applications where a strong non-absorbable suture is desired. For example, ARTHREX™ FIBERWIRE™ is a family of strong braided sutures particularly useful in orthopedic soft tissue applications. FIBERWIRE™ includes a blend of an ultra high molecular weight polyethylene multi-filament core with a braided polyester jacket. However, these sutures are anchored to tissue utilizing anchoring devices and knots and to that extent do not have the advantages of self-retaining sutures. Applicants disclose herein self-retaining braided sutures and methods of manufacturing and using self-retaining braided sutures.

### Self-Retaining Braided Sutures

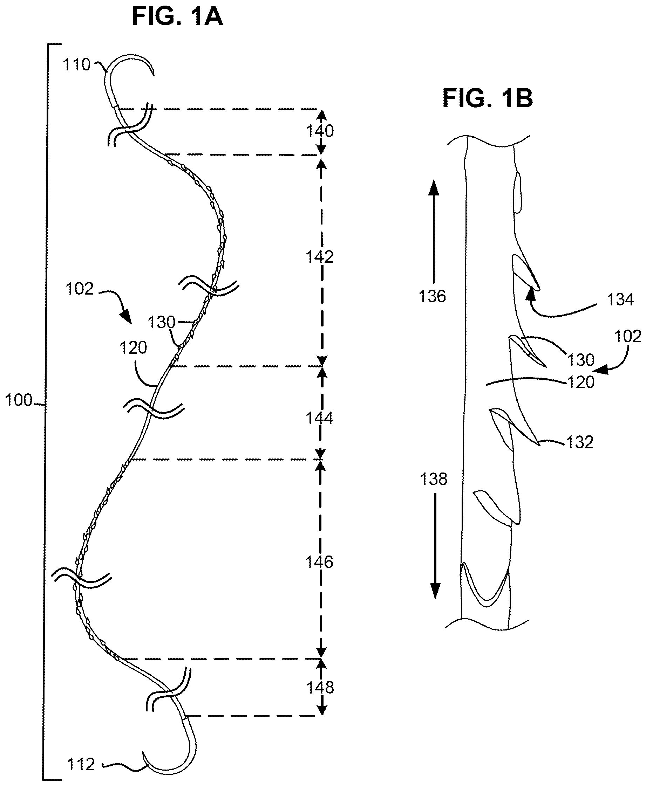

FIG. 1A illustrates a bidirectional self-retaining braided suture system 100. Self-retaining suture system 100 comprises needles 110, 112 attached to self-retaining braided suture 102. Self-retaining braided suture 102 includes a plurality of retainers 130 distributed on the surface of a braided suture 120. In lead-in region 140 of braided suture 120 there are no retainers 130. In region 142 of braided suture 120 there are a plurality of retainers 130 arranged such that the suture can be deployed in the direction of needle 110 but resists movement in the direction of needle 112. In transition region 144, there are no retainers 130. In region 146, there are a plurality of retainers 130 arranged such that the suture can be deployed in the direction of needle 112 but resists movement in the direction of needle 110. In lead-in region 148 of braided suture 120 there are no retainers 130. A break is shown in each of regions 140, 142, 144, 146 and 148 to indicate that the length of each region may be varied and selected depending upon the application for which the suture is intended to be used. Although a bidirectional self-retaining suture system 100 is illustrated, the present invention includes self-retaining suture systems of a wide variety of retainer and needle configurations described above. Likewise the configuration of each of needles 110 and 112 can be any of the range of different surgical needles developed for use in different applications. Needles 110 and 112 may have the same configuration or different configurations.

FIG. 1B illustrates a magnified view of self-retaining suture 102 in region 142. As shown in FIG. 1B, a plurality of retainers 130 is distributed on the surface of braided suture 120. The affixation of self-retaining sutures after deployment in tissue entails the penetration of retainer ends into the surrounding tissue resulting in tissue being caught between the retainer and the suture body. The inner surface of the retainer that is in contact with the tissue that is caught between the retainer 130 and the braided suture 120, herein referred to as the "tissue engagement surface" or "inner retainer surface," can be adapted to better engage the tissue. As illustrated in FIG. 1B, each retainer 130 has a tip 132 and tissue retainer surface 134. When self-retaining suture 102 is moved in the direction of arrow 136, retainer 130 lies flat against the body of braided suture 120. However, when self-retaining suture 102 is moved in the direction of arrow 138, tip 132 of retainer 130 engages tissue surrounding braided suture 120 and causes retainer 130 to fan out from braided suture 120 and engage the tissue with face 134 thereby preventing movement of the suture in that direction.

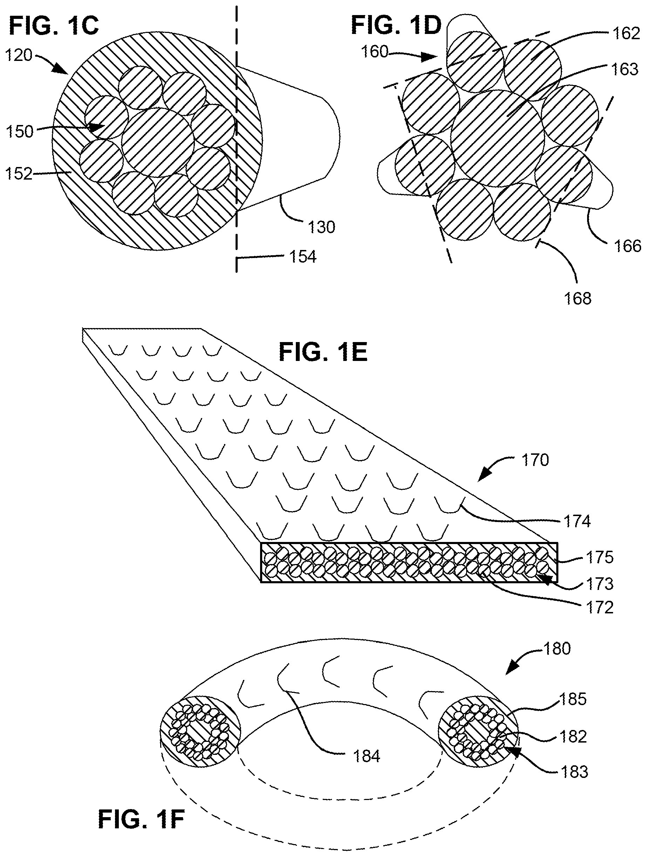

FIG. 1C shows a cross-sectional view of braided suture 120. As shown in FIG. 1C, braided suture 120 includes a braided core 150 and a sheath 152. Because the retainers 130 are formed on the surface of braided suture 120, the retainers are made, in this embodiment, entirely of the material of sheath. Line 154 shows the depth to which the base of retainer 130 is cut into braided suture 120. Accordingly, the material of sheath 152 is selected to have properties which are advantageous to the creation, elevation, deployment and function of retainers 130. Furthermore, in specific embodiments of the present disclosure, retainers 130 include little or none of the material of the braided core 150. Thus, material of the braided core 150 is selected to have properties which are advantageous to the mechanical properties of the suture.

For example, in certain embodiments material of sheath 152 may be selected to have a larger plastic deformation zone (also known as work hardening zone) i.e. more ability to undergo plastic (permanent) deformation than the material of core 150. This permits retainers 130 to be elevated (bent away) from braided suture 120 and plastically deformed into the elevated position away from braided suture 120. Furthermore, in certain embodiments, the material of core 150 is selected to have greater tensile strength and/or elasticity, and preferably greater flexibility than material of sheath 152 which permits braided suture 120 to have a greater tensile strength and/or resistance to breakage than if the suture was entirely made of the material of sheath 152. In alternative embodiments, retainer 130 may comprise the material of sheath 152 and also some portion of the material of core 150 or another non-sheath material. In such embodiments, the materials are selected such that the braided properties of the materials in the retainer permit or enhance the function of the retainer such as by facilitating elevation of the retainer 130.

FIG. 1D shows an alternative cross-sectional view of braided suture 160. As shown in FIG. 1D, braided suture 160 includes a braid of fibers 162. The braided 160 is not covered with a sheath. Instead, the retainers 166 are formed on the surface of braided suture 160. The retainers are made, in this embodiment, entirely of the material of the outermost fibers 162 of braided suture 160. Line 168 shows the depth to which the base of retainer 166 is cut into the fiber 162 of braid 160. Accordingly, the material of fibers 162 is selected to have properties which are advantageous to the creation, elevation, deployment and function of retainers 166 and also to the strength of braided suture 160. In certain embodiments there may be a core fiber 163 or fibers around which other fibers 162 are wound. This fiber may be selected to have properties which are advantageous to the mechanical properties of the suture without regard to the formation of retainers.

For example, in certain embodiments material of external fibers 162 may be selected to have a larger plastic deformation zone (also known as work hardening zone) i.e. more ability to undergo plastic (permanent) deformation than the material of core 163. This permits retainers 166 to be elevated (bent away) from braided suture 160 and be plastically deformed into the elevated position away from braided suture 160. Furthermore, in certain embodiments, the material of core fiber 163 is selected to have greater tensile strength and/or elasticity, and preferably greater flexibility than material of external fibers 162 which permits braided suture 160 to have a greater tensile strength and/or resistance to breakage than if the suture was entirely made of the material of external fibers 162.

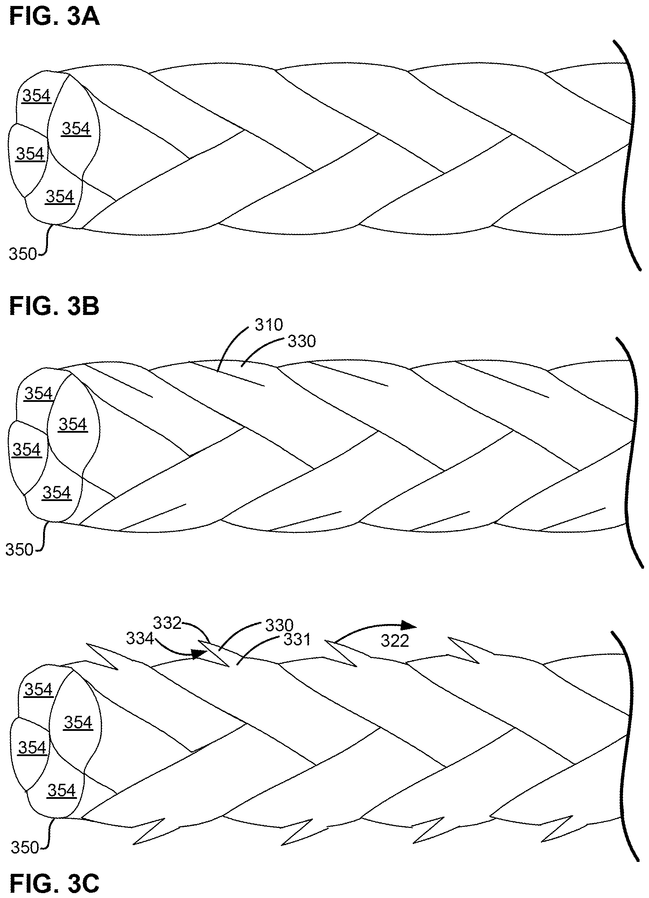

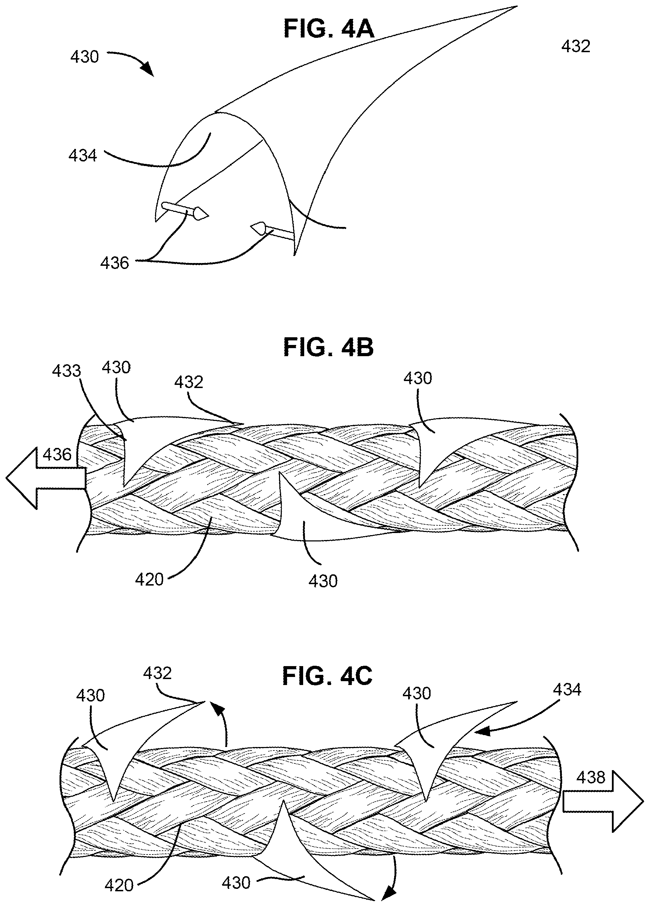

As shown in FIGS. 1E and 1F, fibers may be formed into shapes other than surgical suture threads. For example, as shown in FIG. 1E, fibers may be woven into a two dimensional structure such as a flat ribbon or sheet. Two dimensional sheets and ribbons may be particularly useful for supporting tissues and/or closing openings in tissue. FIG. 1E shows a ribbon structure 170. Ribbon structure is planar or substantially rectangular in cross-section. Ribbon structure 170 comprises a plurality of fibers 172 braided into a ribbon 173. As shown in FIG. IE, retainers 174 may be formed in a sheath 175 applied to the ribbon 173. In alternative sheathless embodiments (not shown), the fibers 172 themselves may be provided with retainers 174 as described herein with respect to a braided suture (See, e.g. FIGS. 3A-3G and accompanying text). In such embodiments, retainers 174 may be formed on the fibers 172 of the braided ribbon 173 before or after braiding. In other alternative embodiments (not shown), separately-formed retainers may be added to the braided ribbon 173 (See, e.g. FIGS. 4A-4C and accompanying text).

As shown in FIG. IF, fibers may also be woven into 3-dimensional structures such as rings or tubes. Particular 3-dimensional structures may be braided depending upon the needs of a particular surgical procedure. FIG. 1F shows a circular structure 180. The circular structure 180 is shown in partial section to illustrate the interior of the structure -- the dashed lines illustrate the outline of the second half of the circular structure 180. Circular structure 180 comprises a plurality of fibers 182 woven into a tube 183 and formed into the circular shape of circular structure 180. The tube 183 of braided fibers 182 is one example of a particular 3-dimensional structure which may be braided depending upon the needs of a particular surgical procedure. As shown in FIG. IF, retainers 184 may be formed in a sheath 185 applied to the braided plurality of fibers 182. In alternative sheathless embodiments (not shown), the fibers 182 themselves may be provided with retainers 184 in the same manners as described herein with respect to a braided suture (See, e.g. FIGS. 3A-3G and accompanying text). In such alternative embodiments, retainers 184 may be formed on the fibers 182 of the tube 183 before or after braiding. In other alternative embodiments (not shown), separately-formed retainers may be added to the braided tube 183 (See, e.g. FIGS. 4A-4C and accompanying text).

### Retainer Formation and Elevation

Braided suture threads described herein may be produced by any suitable method, including without limitation, injection molding, stamping, cutting, laser, extrusion, and so forth. In some embodiments, the braided suture may have a sheath covering the braid. The sheath may be applied by extrusion, dipping, spraying, coating and so forth. Braided sutures may be manufactured or purchased for the suture body, and the retainers can be subsequently formed onto the suture body. One-way of forming retainers is to make one or more angled cuts into the surface of the braided suture - either into the sheath of a braided suture (See FIG. 1C) or into the outermost fibers of a braided suture (see FIG. ID). A retainer formed from more than one angled cut, e.g., two angled cuts, will have an underside that is characterized by more than one slope, e.g., two slopes, relative to the center axis of the suture, when viewed in a cross-section along the length of the suture. Alternatively, the retainers may be formed by deforming the sheath or outermost fibers mechanically, chemically and/or thermally by coining, stamping, pinching molding and so forth. Alternatively, retainers may be added to the surface of the braided suture and attached to the braided suture, mechanically or by adhesive or welding or other methods of joining the retainer to the suture.

With respect to forming retainers by cutting, the retainers may be hand-cut, laser-cut, electrical-discharge-cut (EDM), plasma cut, chemically-etched or mechanically machine-cut using blades, cutting wheels, grinding wheels, and so forth. During cutting either the cutting device or the suture may be moved relative to the other, or both may be moved, to control the size, shape and depth of the cut. Particular methods for cutting barbs on a suture are described in U.S. Patent Application Serial No. 09/943,733 titled "Method Of Forming Barbs On A Suture And Apparatus For Performing Same" to Genova et al., and U.S. Patent Application Serial No. 10/065,280 titled "Barbed Sutures" to Leung et al.

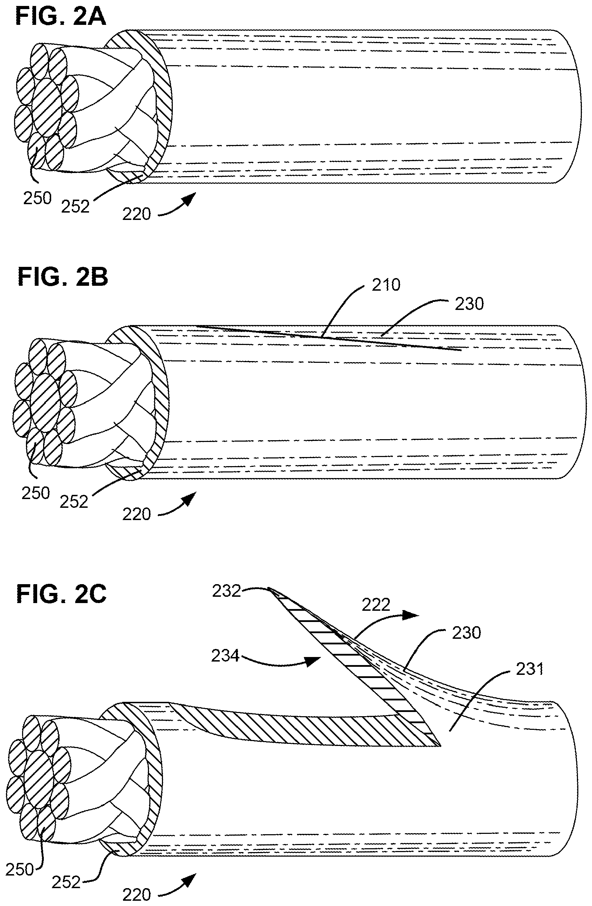

Referring now to FIGS. 2A, 2B and 2C where an exemplary process for making a retainer 230 by cutting into the surface of a braided suture 220 is provided. As shown in FIG. 2A braided suture 220 comprises braided core 250 and a sheath 252. Braided suture 220 may be formed by any method known in the art for making a braided suture having a sheath over a braided core each having the properties required for the function of the material in the suture fiber or thread. One suitable method is extrusion of material over a preformed braided core filament. Other methods of forming a sheath on a preformed braid may also be utilized including, without limitation, dip coating, spray coating, curtain coating and/or chemical deposition (for example, chemical vapor deposition CVD). Another method is insertion of the braided core into a tube of sheath material and then shrinking (for example by heating) the tube of sheath material so that it secured mechanically to the core.

As shown in FIG. 2B a retainer 230 may be formed on braided suture 220 by making an angled cut 210 into the sheath 252 of braided suture 220. Cut 210 can be made using any of a wide range of technologies as discussed above. The depth of cut is preferably selected such that cut 210 is entirely within material of sheath 252, as shown in FIG. 2B, and does not penetrate into core 250. The cut depth selected is a compromise between retainer strength and tensile strength of the suture. A cut too deep may cause the suture to be reduced in strength and a cut that is too shallow may cause the retainer not to be strong enough to effectively engage tissue. Generally the cut depth will be selected to be from 10% to 50% of the diameter of the braided suture. More preferably the cut depth will be from 20% to 30% of the diameter of the braided suture.

In a braided suture 220 as shown in FIG. 2B, a primary contribution to the tensile strength of the suture is the tensile strength of the core. Thus, the depth of cut is preferably selected so as not to cut into the core. The thickness of the sheath may be increased or decreased to enable cuts having a greater or lesser depth. The selection of the depth of the sheath should be made to allow the resulting retainers to be sufficiently strong so as to effectively engage the tissue.

In some cases, the depth of the cut used to form a retainer may be selected so that the depth of cut is approximately equal to the depth of the sheath layer. In such cases the resulting braided self-retaining suture will depend for its tensile strength entirely upon the tensile strength of the core. By cutting entirely or almost entirely through the sheath layer, the flexibility of the suture may also be increased. The pattern of retainers on the suture may be, and in one embodiment of the invention is, selected so as to radially offset adjacent retainers so as to prevent the retainers from pulling away from the core. However, the braided core provides significant surface area for bonding between the braided core and the sheath to prevent delamination. In an alternative embodiment the depth of the cut used to form a retainer may be selected so that the depth of cut is somewhat larger than the depth of the sheath layer. In such case the retainers are formed in said sheath and partially in said braided core.

In order for retainer 230 to effectively engage tissue after deployment, tip 232 is preferably elevated above the surface of braided suture 220. As shown in FIG. 2B, retainer 230 may still lay flat against the surface of braided suture 220 after cut 210 has been made in material of sheath 252.As shown in FIG. 2C, after the retainer cutting step of FIG. 2B, retainer 230 is mechanically bent away from the body of braided suture 220 in the direction shown by arrow 222. Tip 232 is moved above the surface of braided suture 220 and tissue engagement surface 234 is exposed. The elevation of retainer 230 can be achieved by a number of mechanisms. In a simple example, a cutting blade is used to form cut 210 and the cutting blade is then removed from cut 210 in a manner that bends retainer 230 away from the body of braided suture 220. In an alternative example, the retainer is mechanically elevated by a device other than the blade.

If material of sheath 252 is too elastic, retainer 230 will spring back to the retainer's previous position flush with the surface of braided suture 220 (as shown by the dotted line) after elevation of the retainer. This is also the case if the material does not have the ability to undergo permanent deformation. Thus, in accordance with a specific embodiment of the present invention, material of sheath 252 is selected such that it is sufficiently plastically deformable (has sufficient plasticity) that after retainer 230 has been moved away from braided suture 220, sheath material remains in its new deformed shape with the tip 232 of retainer 230 substantially elevated above the surface of braided suture 220 and tissue engagement surface 234 exposed. To put it another way, the retainer is plastically deformed into an elevated configuration. The material of sheath 252 is selected such that the mechanical movement of tip 232 of retainer away from braided suture 220 is sufficient to plastically deform the region 231 of material 252 at the base of retainer 230 causing it to take on a new permanent shape. Thus, elevating the retainers plastically deforms the retainers into an elevated configuration. However, because such plastic deformation would be undesirable in the suture as a whole, the material of core 250 is generally selected to have significantly lower plasticity and significantly higher elasticity and/or tensile strength than the material of sheath 252.

It is also possible, by appropriate selection of fiber or thread or filament materials, to create a self-retaining suture in which the retainers elevate without requiring external mechanical intervention or in which the retainers self-elevate to augment the effects of mechanical intervention to produce a greater combined elevation. This is advantageous as it reduces the need for mechanical elevation which is time consuming, expensive and has the potential for weakening the retainers. Depending upon the materials chosen, the retainers can be made to elevate when manufactured or upon insertion into tissue during a procedure. For example, a sheath material may be selected such that during the manufacture of the braided suture, tension is created in the sheath material. This can be achieved, for example by selecting a sheath material that shrinks after the braid has been coated with the sheath while selecting a core braid that does not shrink or shrinks less than the sheath material. For example, polyesters such as polyglycolide or polyglycolide copolymers with epsilon-caprolactone or L-lactide will shrink upon heating to about 90-120°C. Thus, a sheath of this material may be selectively shrunk relative to the core prior to forming retainers. Alternatively a residual stress may be left in the sheath material compared to the core material by the extrusion and drawing process. When a cut is made through the sheath material, the tension in sheath material causes the sheath to contract as shown by arrows. Contraction of the sheath causes the retainers to self-elevate. Thus, in this example, the retainers elevate themselves without mechanical intervention directly upon creating a cut in the surface of the filament. In other embodiments the sheath can be caused to shrink and the retainers elevated by exposure to, for example heat, pH or light. In such cases, the retainers may be elevated before, during or after implantation in the tissue of the patient as required by the application.

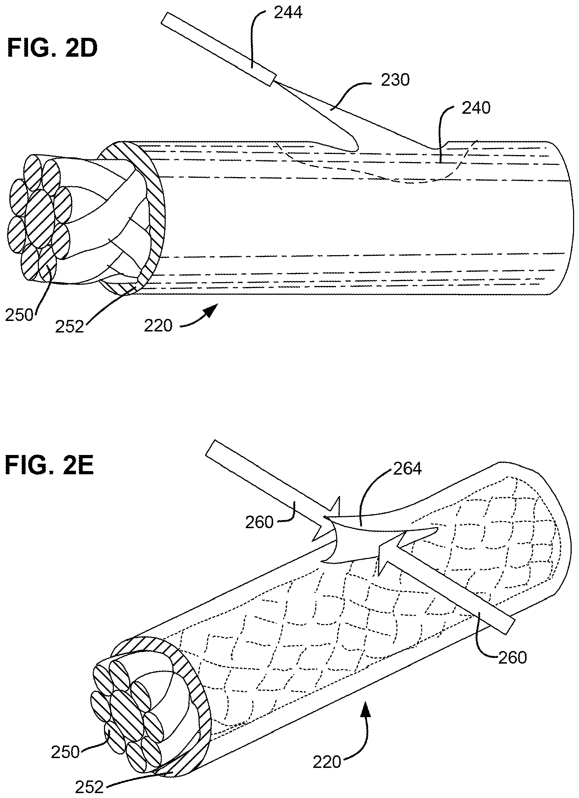

In other embodiments, a retainer may be formed by a process other than cutting into the sheath of the suture fiber or thread. For example, as shown in FIG. 2D a retainer 230 is formed by melting the material of sheath 252 in region 240 and then drawing material out of suture fiber or thread 220 with device 244 and then cooling the material. In this embodiment the material of sheath 252 is selected such that it may be melted and manipulated without disrupting the tensile strength of the core. In alternative embodiments a preformed retainer may be affixed mechanically, adhesively or by melting to the sheath. The sheath material is in this embodiment selected to enhance the affixation of the retainer to the suture fiber or thread and retention of the retainer by the suture fiber or thread. In another embodiment molten material is formed onto the sheath in the shape of a retainer and the molten material fuses with the sheath material. The sheath material in this case is selected to enhance the adhesion or fusion with the externally applied molten material. In some cases the molten material may be the same material as the sheath.

FIG. 2E is a perspective view illustrating an alternative method to create a retainer of a self-retaining braided suture comprising braided core 250 and sheath 252 in accordance with an embodiment of the present invention. As shown in FIG. 2E, a retainer may be formed, preferably in the sheath, by using a mechanical device 260. Mechanical device 260 may be, for example, a pinching, coining, or pressing device. Mechanical device 260 may be in the form, for example of rollers, a press, vice and/or any other mechanical device that exerts a mechanical force. Mechanical device 260 exerts forces on either side of sheath 252 to squeeze the material of sheath 252 and plastically deform the material so that it is forced into the shape of a retainer 264. The shape of retainer 264 will be determined by the device 260 used to make it as well as how that device is employed. In some embodiments, the suture 220 may be heated during retainer formation to a temperature that facilitates plastic deformation of the sheath 252.

Retainers may also be formed on braided sutures not having a sheath. However, in such cases, the cut position and depth of cut should be selected so as not to cut entirely through a fiber of the braid. As the fiber diameter will be significantly less than the total braid diameter, the depth of cut will be significantly smaller than for e.g. a monofilament suture of the same diameter. Thus retainers formed directly on the surface of a braid will ultimately be significantly smaller than could be achieved on a braided suture with a sheath or on a monofilament. However the number of retainers also factors into the holding strength of a self-retaining suture, thus, creating a large number /high density of smaller retainers can generate sufficient holding strength.

FIGS. 3A-3C illustrate method steps in the creation of retainers on fibers of a braid. As shown in FIG. 3A braided suture 350 comprises a braid of four fibers 354. Braided suture 350 may be formed by any method and using standard equipment known in the art for making a braided suture having the strength, flexibility and diameter required. The braided suture may be braided from one type of fiber or multiple types of fiber and can be braided with or without a core depending upon the application.

As shown in FIG. 3B a retainer 330 may be formed on braided suture 350 by making a cut 310 into a fiber 354 of the braided suture 350. Cut 310 can be made using any of a wide range of technologies as discussed above. The depth of cut has been selected such that a particular cut 310 is entirely within material of a single fiber 354 and does not penetrate entirely through the fiber 354. The cut depth selected is a compromise between retainer strength and tensile strength of the suture. A cut that is too deep may cause the suture to be reduced in strength and a cut that is too shallow may cause the retainer not to be strong enough to effectively engage tissue. Generally the cut depth will be selected to be from 10% to 50% of the diameter of a fiber 354 of the braided suture 350.

In order for retainer 330 to effectively engage tissue after deployment, tip 332 is preferably elevated above the surface of braided suture 350. As shown in FIG. 3B, retainer 330 may still lay flat against the surface of braided suture 350 after cut 310 has been made in fiber 354. As shown in FIG. 3C, after the cutting step of FIG. 3B, retainer 330 is mechanically bent away from the body of braided suture 350 in the direction shown by arrow 322. Tip 332 is moved above the surface of braided suture 350 and tissue engagement surface 334 is exposed. The elevation of retainer 330 can be achieved by a number of mechanisms. In a simple example, a cutting blade is used to form cut 310 and the cutting blade is then removed from cut 310 in a manner that bends retainer 330 away from the body of braided suture 350 at region 331. In an alternative example, the retainer is mechanically elevated by a device other than the blade.

If material of fiber 354 is too elastic, retainer 330 will spring back to the retainer's previous position flush with the surface of braided suture 320 (as shown by the dotted line) after elevation of the retainer. This is also the case if the material of fiber 354 does not have the ability to undergo permanent deformation. If all the fibers 354 of the braided suture 350 are the same, then the choice of material for the fibers 354 is tempered by the structural needs of the suture and the properties required for the retainer. The material should be is selected such that the mechanical movement of tip 332 of retainer away from braided suture 320 is sufficient to plastically deform the region 331 of material 352 at the base of retainer 330 causing it to take on a new permanent shape.

In certain embodiments, retainers may be formed on a braid in a process subsequent to manufacturing the braid. Retainers may be hand-cut, laser-cut, or machine-cut using blades, cutting wheels, grinding wheels, and so forth. During cutting either the cutting device or the suture may be moved relative to the other, or both may be moved, to control the location, size, shape, angle, and depth of cut. Particular methods for cutting barbs on filaments are described in U.S. Patent No. 7,225,512 titled "Method Of Forming Barbs On A Suture And Apparatus For Performing Same" to Genova et al., and U.S. Patent Application Serial No. 10/065,280 titled "Barbed Sutures" to Leung et al.

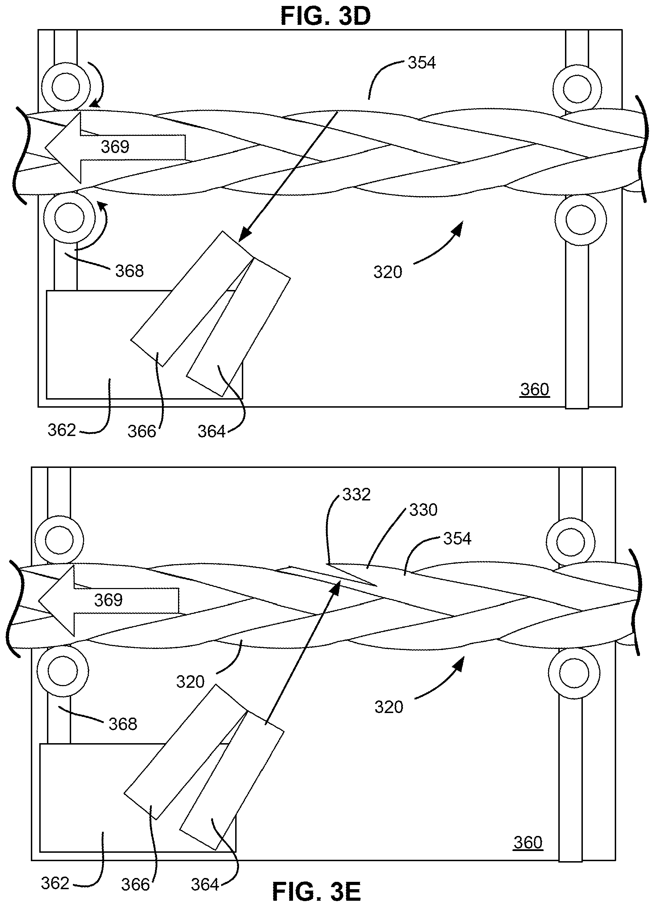

When retainers are formed on a braid without a sheath, steps are preferably taken to identify locations for the retainers and register the braid prior to cutting the retainers in order to enhance the creation of the retainers. Preferably this identification and registration permits the creation of retainers with a depth that is a significant fraction of the diameter of a fiber while avoiding cutting entirely though fibers to achieve enhanced tensile strength. Moreover, as the retainer size is significantly smaller than for monofilaments of the same diameter, it is preferable to use cutting techniques suitable for creating small features, for example, EDM and laser cutting. One way to form retainers on a braid is by cutting into the braid as illustrated in FIGS. 3D and 3E.

Referring now to FIGS. 3D and 3E where an exemplary process for cutting a retainer 330 on a braided suture 320 with a cutting machine 360 is provided. As shown in FIG. 3D braided suture 320 consists of a plurality of fibers 354. In order to form the retainer 330 in the appropriate position relative to fiber 354 it is first necessary to identify the position of the fiber and advance and or rotate the braid 320 into the correct position in which fiber 354 is appropriately positioned relative to cutting mechanism 300. As shown schematically in FIG. 3D, braided suture 320 is advanced (indexed) in the direction of arrow 369 relative to a sensor 366 and cutting mechanism 364 by an indexing mechanism 368. Braided suture 320 may be advanced by an indexing mechanism 368 that includes devices such as rollers or drums or other actuators suitable for moving fiber with the accuracy necessary to position fiber 354 relative to the sensor 366 and cutting device 364. The braid may also be rotated/twisted by a suitable mechanism during the cutting process so as to distribute the retainers around the circumference of the braid. A controller 362 receives input from the sensor 366 and controls indexing mechanism 368 to correctly position fiber 354 relative to cutting device 364. Alternatively, or additionally, controller 362 may control the position of cutting location (aim) of cutting device 364. For example, cutting device 364 may be a laser device which can be targeted at a location on fiber 354 by an optical means. Sensor 366 monitors the braided suture 320 as braided suture 320 moves in the direction of arrow 369. Sensor 366 is preferably a contactless optical or other electrical sensor system that is capable of identifying the position of the fibers 354 of braided suture 320 as it passes through cutting machine 360. The fiber recognition system of sensor 366 may be assisted by incorporating fibers of different colors in braided suture 320. When sensor 306 detects that a fiber 354 is correctly registered relative to cutting device 364 the movement of braided suture 320 may be stopped and the cutting process conducted.

As shown in FIG. 3D and 3E the operation of cutting device 364 is synchronized with the indexing mechanism 368 for advancing the braided suture 320 by controller 362. With braided suture 320 registered and cutting device 364 targeted, cutting device 364 is operated to form a retainer 330. Where cutting device 364 is an EDM or laser device, material of the fiber 354 is ablated/vaporized from the fiber to form the shape of retainer 330. The shape of the retainer 330 may be controlled using a mask or by directional control of a cutting beam. In certain embodiments, the shape of retainer 330 may be modified based on the dimensions of the particular fiber 354 registered for cutting. For example, it may be desirable that cutting device 364 leaves a certain minimum cross-section in fiber 354. Thus, the operation of cutting device 364 may be adjusted by controller 362 in response to information from sensor 366 so as to adjust the shape/size of retainer 330 based upon the amount of material actually present at a particular point in a fiber 354. This provides additional control by adjusting the cut parameters based upon the measured dimensions of the braided suture 320 and fiber 354 to deal with manufacturing variations from one region to another region. In some embodiments, there may also be a separate elevation step to enhance the elevation of the tip 332 of retainer 330 above fiber 354. This elevation may be achieved using cutting device 364 or a separate elevating component or machine. The elevation may also be achieved in a separate process or in some case automatically because of properties of the material of braided suture 320.

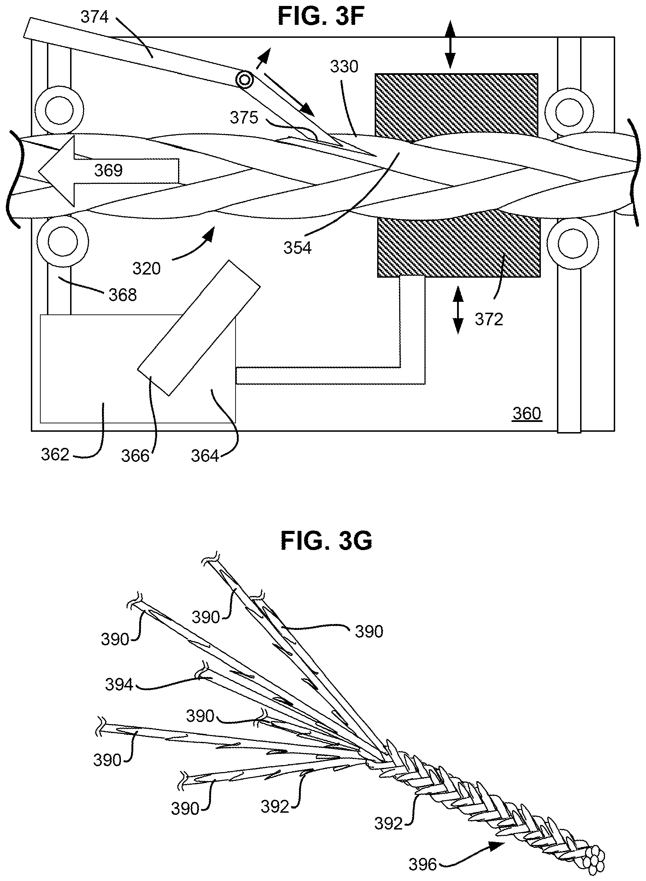

FIG. 3F shows an alternative embodiment for making the braided suture in which a mechanical cutting device 374 is utilized. Mechanical cutting device may be for example, a blade, rotating blade or vibrating blade, fluid jet or other mechanical cutting device of sufficient accuracy. As previously described, the mechanical cutting device 374 is synchronized with the indexing mechanism 368 for advancing the braided suture 320 by controller 362. With mechanical cutting a support vice 372 may be desirable to control movement of the suture 320 during cutting. The support vice 372 may be static or may be synchronized to move in and out by controller 362 to support suture 320 only during cutting. With braided suture 320 registered and cutting device 374 is operated to form a retainer 330. As shown in FIG. 3F, cutting edge 375 of cutting device 374 is advanced into the correct location on a fiber 354 to cut and elevate the retainer 330 in one step. The shape of the retainer 330 may be controlled by directional control of a cutting device 374. In certain embodiments, the shape of retainer 330 may be modified based on the dimensions of the particular fiber 354 registered for cutting. For example, it may be desirable that cutting device 374 leaves a certain minimum cross-section in fiber 354. Thus, the operation of cutting device 374 may be adjusted by controller 362 in response to information from sensor 366 so as to adjust the shape/size of retainer 330 based upon the amount of material actually present at a particular point in a fiber 354. This provides additional control by adjusting the cut parameters based upon the measured dimensions of the braided suture 320 and fiber 354 to deal with manufacturing variations from one region to another region. In some embodiments, there may also be a separate elevation step to enhance the elevation of the tip 332 of retainer 330 above fiber 354. The elevation may also be achieved in a separate process or in some case automatically because of properties of the material of braided suture 320.

In other embodiments, a retainer may be formed on a fiber before creation of a braid. For example, as shown in FIG. 3G six fibers 390 have retainers 392 formed on their surface in a conventional manner. Particular methods for cutting retainers on filaments are described in U.S. Patent No. 7,225,512 titled "Method Of Forming Barbs On A Suture And Apparatus For Performing Same" to Genova et al., and U.S. Patent Application Serial No. 10/065,280 titled "Barbed Sutures" to Leung et al.

The six fibers 390 bearing retainers 392 are then braided around core fiber 394 using conventional braiding machinery. Braiding generates a braided suture 396 having some of the retainers 392 on the surface. Different braiding patterns may be used with or without a core. Fibers may also be braided into 2- dimensional or 3-dimensional shapes, for example, ribbons, sheet, rings, etc. Only fibers appearing on the surface of the final braided suture should have retainers formed on the surface. As previously discussed, the fibers having retainers may be made of a different material than the fibers not having retainers and/or core fiber.

In some embodiments, braided suture 396 may be woven in a fashion so that the fibers on the external surface of braided suture 396 are made of a different material than the fibers interior to the braided suture 396. In such case the exterior material can be selected based on the needs of the retainers and the internal fiber material can be selected based upon the needs of the suture. Alternatively, particular fibers may be made of different materials and those fibers selected for creation of the retainers. In such cases, the fibers should be identifiable by the device making the retainers such as by color or size.

In alternative embodiments a preformed retainer may be affixed mechanically, adhesively or by melting to the braid. The braid material is in this embodiment selected to enhance the affixation of the retainer to the suture and retention of the retainer by the suture. In another embodiment, molten material is formed onto the braid in the shape of a retainer and the molten material fuses with the braid material. The braid material in this case is selected to enhance the adhesion or fusion with the externally applied molten material. In some cases the molten material may be the same material as the braid.

FIG. 4A is a perspective view shows an example of a retainer that may be added to a braided suture 420 to form a self-retaining braided suture in accordance with an embodiment of the present disclosure. As shown in FIG. 4A, a retainer 430 is formed separately from a braided suture, by for example, molding, stamping or coining for later attachment to a braided suture thread. The retainer 430 has a tissue engagement surface 434 for trapping tissue between the retainer 430 and a braided suture or a fiber to the braided suture. Each retainer 430 has a tip 432 and tissue retainer surface 434. Tip 432 is configured to penetrate tissue when retainer 430 is moved in the direction of tip 432. The opposite end/base 433 of retainer 430 is designed to lie close to a suture thread so that the retainer 430 causes little resistance to tissue when travelling in the direction of the base 433 of the retainer 430. Retainer 430 has mechanical devices 436 to engage a braided suture. As shown in FIG. 4B suitable mechanical devices may include barbed pins, loops, hooks and the like which engage the fibers of a braided suture without compromising the strength of the braided suture. Alternatively or additionally, retainer 430 may be secured to the by and adhesive, welding or other bonding technology.

FIG. 4B is a perspective view of a self-retaining braided suture including a plurality of the retainers of FIG. 4A in accordance with an embodiment of the present disclosure. FIG. 4B shows a section of a braided suture 420 to which a plurality of retainers 430 have been added. Retainers 436 (FIG. 4A) have engaged braided suture 420. Base 433 of retainers 430 lies close to the surface of braided suture 420 so that the retainer 430 causes little resistance to tissue when travelling in the direction of the base 433 of the retainer 430 (direction of arrow 436). When self-retaining suture braided 420 is moved in the direction of arrow 436, retainer 430 lies flat against the body of braided suture 420. However, as shown in FIG. 4C, when self-retaining braided suture 420 is moved in the direction of arrow 438, tip 432 of retainer 430 engages tissue surrounding braided suture 420 and causes retainer 430 to fan out from braided suture 420 and engage the tissue with face 434 thereby preventing movement of the suture in that direction. The affixation of self-retaining sutures after deployment in tissue entails the penetration of retainer ends into the surrounding tissue resulting in tissue being caught between the retainer and the suture body.

### Methods Of Making Self-Retaining Braided Sutures

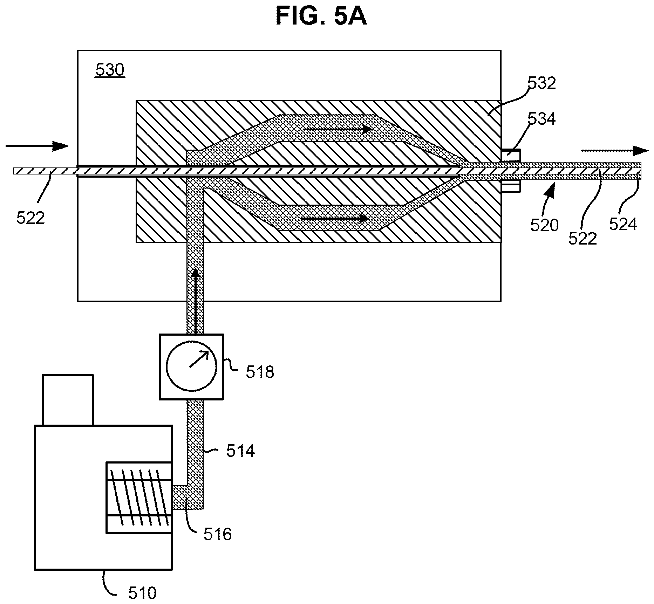

As described above, a braided suture can be made in many different ways. In accordance with one embodiment of the invention, a sheath is applied to a braided core to generate a braided suture. FIG. 5A illustrates one method by which the coating may be achieved U.S. Patent 6,183,499 titled "Surgical Filament Construction" to Fisher et al.

As shown in FIG. 5A, satellite extruder 510 heats, melts and extrudes a material 516 along conduit 514 to extruder 530. Metering pump 518 on conduit 514 controls the flow of material 516 to main extruder 530. Material 516 is provided to extrusion die 532 within extruder 530. A preformed braided core 522 is drawn through extrusion die 532. The rate of supply of sheath material 516 and the rate of movement of braided core 522 are controlled so that sheath material 516 is evenly coated on the core 522 in the desired cross-section (as determined by the cross-section of the extrusion nozzle 534. The finished braided suture 520 comprising braided core 522 and a sheath 524 of material 516 may be quenched, tempered and drawn and then wound onto a drum.

In certain embodiments, braided core 522 cannot be drawn. Indeed, it may not be necessary or desirable to draw finished suture 520 (including core 522) after forming the sheath 524. In some embodiments, for example, fibers for making a braided core 522 are extruded and drawn and the fibers are subsequently braided to form braided core 522. The braided core 522 cannot be drawn any further. Sheath material 516 is then extruded over the braided core 522 (as shown in FIG. 5A). However the finished suture 520 is not drawn after extrusion of the sheath. In some embodiments, the sheath material 516 may be the same polymer as the braided core 522. The resulting suture 520 has a core 522 and sheath 524 of the same polymer, however, the core material has different physical properties than the sheath material because the core fibers were drawn before braiding and the sheath material was not drawn after extrusion.

Many different braided threads and or sutures may be used as the braided core 522 of a self-retaining braided suture in accordance with embodiments of the present invention. For example, core 522 may be a conventional braided suture material. For example, braided sutures such as VICRYL™, SURGICRYL™, BIOVEK™, VISORB™, POLYSORB™, SURGISORB™, and DEXON™ may be provided with a biodegradable sheath suitable for the formation of retainers and the resulting self-retaining braided suture will be useful in applications where it is desirable to have a self-retaining absorbable suture which retains its tensile strength for a period of three to four weeks and is completely absorbed by hydrolysis in a period of ten weeks. These sutures are manufactured using braided polyglycolic acid (PGA) material and may be coated with a polycaprolactone and calcium stearate sheath into which retainers may be cut. Retainers may be formed in the sheath layer to create self-retaining braided sutures in accordance with embodiments of the present invention.

In another example braided sutures such as ARTHREX™ FIBERWIRE™ may be provided with a nonbiodegradable or non-absorbable sheath suitable for the formation of retainers and the resulting self-retaining braided suture will be useful in applications where a strong non-absorbable suture is desired. FIBERWIRE™ includes a blend of an ultra high molecular weight polyethylene multi-filament core with braided polyester jacket and may be coated with extruded polyester into which retainers may be cut. Retainers may be formed in the sheath layer to create self-retaining braided sutures in accordance with embodiments of the present invention. Preferably, substantial retainers are provided suitable for engaging soft tissue to approximate e.g. tissue to bone. The retainers are designed to be effective to engage the intended tissues, for example tough tissues, fascia, capsule, meniscus-like structures, glenoid labrum in shoulder, flexor tendons, tendons, rotator cuffs. Typically the suture will tether such tissues to bone or periosteum. Thus, the braided suture may include a suture loop of anchoring device at one end to engage a bone tunnel, bone, periosteum or another stable anatomical feature. Such braided sutures may be used in arthroscopic applications where a strong non-absorbable self-retaining braided suture is desired.

Although extrusion has been illustrated in FIG. 5A, any suitable manufacturing process may be used to form the braided sutures utilized as a stock braid material for embodiments of the present invention. For example, braided sutures may be provided with a sheath by coating it with a polymer or dipping in a polymer or spraying with a polymer. Alternatively the outer fibers of a braided suture may be fused to form a sheath by heating or chemical treatment of the surface fibers of the braided suture thread. Apparatus and methods for forming braided sutures suitable for use in the present invention can be found in U.S. Patent 6,183,499 titled "Surgical Filament Construction" to Fisher et al. and U.S. Patent 6,315,788 titled "Braided Materials And Surgical Articles Made Therefrom" to Roby.

### Braided Suture Configurations For Self-Retaining Braided Sutures

Depending upon the configuration of the braided core, extruders, die, or other manufacturing equipment, a braided suture suitable for creating a self-retaining suture in accordance with embodiments of the present disclosure can be created with a wide variety of different arrangements of different materials. Furthermore, braided sutures can be made using 2, 3, 4 or even more different component materials if necessary or desired for the particular application. Different configurations of braided sutures are useful in specific embodiments of the present disclosure and are described below with respect to FIGS. 5B, 5C, 5D and 5F which show cross-sections and/or different suture configurations. Different configurations of braided sutures are useful in specific embodiments of the present invention and are described below with respect to FIG. 5E which shows a cross-section and/or different suture configuration.

The configuration of the materials in the braided suture will depend upon the characteristics of the materials and the amount of material necessary to fulfill the role of the suture. For example, in one embodiment the material of sheath 524 is chosen to be plastically deformable in order that barbs may be more easily formed and elevated from the surface of the suture. The depth of the sheath may thus be chosen such that the retainers when formed are formed entirely out of the sheath material. Likewise in one embodiment of the present invention, the material of the core 522 is chosen because of its characteristic of tensile strength. The strength of the final braided suture will depend in large part upon the cross-sectional area of core 522. Thus core 522 is desirably as large as possible while providing sufficient amount of sheath material 524 to permit the formation of retainers. The overall diameter of the braided suture is also limited based upon the surgical needs.

As shown in FIGS. 5B and 5C simple braided sutures 520B, 520C comprise a braided core 522B, 522C and a second material as a sheath 524B, 524C over the core. This arrangement of materials in a braided suture can be made by extrusion of the sheath material over the braided core as shown in FIG. 5A. In FIG. 5B, the core material 522B takes up about 25% of the cross-sectional area of suture 520B, with the sheath material 524B taking up 75% of the cross-sectional area. In comparison, in FIG. 5C, the core material 522C and sheath material 524C each take up about 50% of the cross-sectional area. In general, the core material may comprise from 10% to 90% of the total cross-sectional area of the suture. Preferably the core material will comprise from 25% to 90% of the total cross-sectional area of the suture. More preferably the core material will comprise more than 50% of the total cross-sectional area of the suture. Note that the depth of cut for retainers is smaller for suture 520B as illustrated by line B B of FIG. 5B than for suture 520C as illustrated by line C-C of FIG. 5C

FIG. 5D illustrates an alternative suture 520D in which the braided core 522D includes a jacket of fibers 525D braided over a core braid 523D. The braided core can be formed according to any braiding techniques known in the art using conventional machinery. The braided core may include one or more materials and may be treated and or coated in order to enhance adhesion of the sheath 524D to the braided core 522D. FIG. 5D also demonstrates that retainers may be formed in a suture using a curved cut as illustrated by line D-D. By using a curved cut, larger retainers may be formed on the surface of the suture without cutting into the braided core. Other shapes of cut may also be useful depending on the configuration of the core and the sheath, for example a V-shaped cut.

FIG. 5E illustrates another alternative braided suture 520E for use in the present invention. Braided suture 520E is in the form of a ribbon. Braided suture 520E shows that it is not necessary for the core and/or sheath to be circular in cross-section. A ribbon shape is useful for supporting tissues in a manner that provides greater support to the tissue and reduces pull through. The thickness of the sheath can be controlled by the shape of the extrusion die relative to the braided core. As shown in FIG. 5E, the sheath 524E is thicker on the edges of ribbon-shaped core 522E. In this embodiment, it is therefore preferable that the retainers be formed on the edges of braided suture 520E. The maximum cut depth for creating the retainer without cutting into the core 522E is illustrated by line E-E of FIG. 5E. In one embodiment of the invention, retainers are formed by cutting into both the sheath 524E and the core 522E.

FIG. 5F illustrates an alternative embodiment in which the core and sheaths of suture 520F have different shapes. In the embodiment of FIG. 5F, core 522F has a circular cross-section while the sheath 524F has a triangular cross-section. This arrangement provides a greater volume of the sheath material at the apices 526 of the triangle while still allowing the core material to provide a high percentage of the total cross-sectional area of the suture. In this embodiment, the retainers are optionally cut into the apices of the triangular cross section thus making optimal use of the material in the sheath 524F. In addition, the retainer configuration may be selected such that retainers with V-shaped bases are cut into the apices of the triangle. Dashed line F-F illustrates the cut for a V-shaped base of a retainer and illustrates that the cut extends through a greater amount of the sheath 524F than would a straight cut. Methods for making self-retaining suture from suture threads with triangular or other polyhedral cross-section are disclosed in U.S. Patent 5,342,376 titled "Inserting Device For A Barbed Tissue Connector" to Ruff. The arrangement of materials in a braided suture shown in FIG. 5F can be made by co- extrusion of the two materials. The extruder nozzle is selected to have the desired shape. The shape of the cross-section of the suture matches the shape of the extruder nozzle. Alternatively, the suture may be formed as in FIG. 5A and then the sheath material 524 may be formed into the triangular shape by post-extrusion manipulations, such as using rollers to pinch the material into shape and then heating to anneal the polymer into the chosen shape prior to creation of the retainers. Naturally, other geometric arrangements of the materials are possible, for example the sheath may be formed with a square cross-section, pentagonal, hexagonal or other polygonal cross- section.

### Materials For Self-Retaining Braided Sutures

It is an advantage of the present invention that the material of the sheath of the suture thread may have different properties than the material of the core. The material of the sheath may thus be selected to have properties useful for retainer formation, elevation and deployment material and the material of the core may be selected for properties such as strength and flexibility. Suitable materials for the core include many materials that are currently used for making sutures. Suitable non-degradable suture materials for the core material include polyamide (also known as nylon, such as nylon 6 and nylon 6.6), polyester (e.g., polyethylene terephthlate), polytetrafluoroethylenes (e.g., expanded polytetrafluoroethylene), polyether-ester such as polybutester (block copolymer of butylene terephthalate and polytetra methylene ether glycol), 4-hydroxybutyrate, polyhydroxylalkanoate, polyurethane, metals and metal alloys (e.g., stainless steel wire), polypropylene, polyethelene, silk, and cotton. Suitable absorbable materials for the core include polyglycolic acid homopolymer, copolymers of glycolide and ε-caprolactone, copolymers of glycolide and lactide, copolymers of trimethylene carbonate and glycolide with diethylene glycol (e.g., MAXON™, Tyco Healthcare Group), polyhydroxylalkanoates (such as poly(4-hydroxybutyrate) or poly(4-hydroxybutyrate-co-3-hydroxybutyrate)), terpolymer composed of glycolide, trimethylene carbonate, and dioxanone (e.g., BIOSYN™ [glycolide (60%), trimethylene carbonate (26%), and dioxanone (14%)], Tyco Healthcare Group), copolymers of glycolide, caprolactone, trimethylene carbonate, and lactide (e.g., CAPROSYN™, Tyco Healthcare Group).

Suitable materials for a braid or braided core are characterized by high yield strength after drawing and sufficient flexibility to ease handling. One suitable core material is copolymer of glycolide and ε-caprolactone, in a ratio of 50/50 to 95/5. More preferably the ratio of glycolide to ε-caprolactone is between preferably 70/30 to 80/20 and most preferably between 72/28 and 78/22. In some embodiments the core material has an elastic constant (Young's modulus) between 414 MPa and 4140 MPa (60,000 and 600,000 PSI). Preferably the core material has an elastic constant greater than 690 MPa (100,000 PSI). In most embodiments the elastic constant of the core material will be less than 2760 MPa (400,000 PSI). However a core material with a higher elastic constant will be suitable if it has sufficient toughness and flexibility. Conversely a material with a lower elastic constant will be suitable if it has a low yield strength and sufficient toughness and a large plastic deformation zone with a sufficiently high ultimate tensile strength. In some embodiments the core material will have a low plasticity (amount of plastic deformation as a percentage of total deformation before breaking) of 5-70% and 10-100% elongation at break. However, preferably the core material has a plasticity of around 30% and 15-80% elongation at break. Thus, preferably the core material has a higher, greater or larger elasticity (amount of elastic deformation as a percentage of total deformation before breaking), lower plasticity, and/or higher, greater or larger tensile strength than the material of sheath. Most suitable core materials will have an elongation at break of 20-50%. The above elastic constant, plasticity and elongation at break values are for the core material as present in the finished suture (after drawing and/or other treatments).

Some suitable suture materials may also have more than one elastic constant in the tensile curve which can be represented by two differentially sloped linear regions in the stress-strain curve, in such cases the combination of elastic constants are considered. In alternative embodiments, suture having a core made from a material with a large plastic deformation zone can be advantageous in various applications which require permanent deformation to be imposed on the suture during use. In some embodiments, suture made from a material with a high yield strength e.g. elastic suture, can be advantageous in various applications which require compliance.

Because, in some embodiments, the retainers are predominantly formed from the separate material or materials of the sheath, the sheath layer or layers (or outer fibers/jacket of a braid) may incorporate materials that promote the formation, elevation and deployment of the retainers. Materials that are suitable for the sheath/jacket, in some embodiments, are characterized by having a sufficiently small elastic zone and sufficiently higher, greater, or larger plastic deformation zone/plasticity to allow for permanent deformation of barbs into an elevated position during cutting and elevation and low recoil after elevating the barbs. However, because such plastic deformation would be undesirable in the suture as a whole, the material of core is generally selected to have significantly lower plasticity and significantly higher elasticity and/or significantly higher tensile strength than the material of sheath. It is also desirable to select materials for the sheath with low visco-elastic properties, since in such materials, the retainers may recoil over time and such recoil may go undetected or not be evident immediately after barb cutting and elevation. In certain embodiments the material of the sheath material may be selected to have a larger plastic deformation zone/plasticity (also known as work hardening zone) i.e. more ability to undergo plastic (permanent) deformation than the material of the core material. This permits retainers formed from the sheath material to be elevated (bent away) from the suture and permanently deformed into the elevated position away from braided suture. For example, suitable materials for the sheath include Nylon 6,6, polydioxanone, polypropylene, non-drawn polycaprolactone, poly(4-hydroxybutyrate), non-drawn polydioxanone. Materials which are not drawn typically exhibit a larger plastic region than those which have been drawn. A disadvantage of non-drawn materials can be low stiffness. It is advantageous in some embodiments to use non-drawn materials in the sheath and increase their crystallinity post barb-making by annealing thereby obtaining a higher stiffness of the barb. The retainers can be subjected to heat treatment to increase their stiffness and strength e.g. by appropriate annealing cycles (heating to a certain temperature and cooling at a certain rate) using techniques similar to those taught in U.S. Patent No. 5,007,922 titled "'Method Of Making A Surgical Suture" to Chen et al.

Preferably, the sheath/jacket material is also relatively stiff (i.e. the sheath material has a high elastic constant, but a short elastic zone, and a long plastic zone preferably with a large work hardening coefficient) such that the retainers take a large force to plastically deform, but have low recoil and thus remain in the elevated position after deformation. Additionally the sheath material preferably has sufficient flexural strength to prevent barbs from bending backwards during fixation of the suture in the tissues and sufficient strength to prevent barbs from breaking during fixation of the suture in the tissues. In some embodiments the sheath material has a short elastic zone and a high yield strength. Thus, in some embodiments the sheath material has an elongation at onset of yielding (onset of plastic deformation) of less than 10% and more preferably less than 3% elongation. At the same time the sheath preferably has a high work hardening coefficient and large plastic zone. Additionally, the plasticity (amount of plastic deformation as a percentage of total deformation before breaking) of the sheath material is, in some embodiments, higher than the plasticity of the core material. In some embodiments the sheath material has a plasticity which comprises 5-90% of total elongation and an ultimate elongation (elongation at break) of 10-80%. Alternatively the sheath can have a plasticity which comprises 30-80% of the total elongation and an ultimate elongation of 15-60%. Most preferably the plastic zone of the sheath material comprises 60-90% of the ultimate elongation.

The material of the sheath/jacket in some embodiments also preferably has a high strain-hardening exponent (also known as work-hardening coefficient). Most materials with a distinctive plastic zone have a strain-hardening exponent of 0.1 - 0.5. Many materials with a low strain-hardening exponent (tending towards "perfect plastic") are not desirable as a sheath material due to the inability to withstand excess stress post yielding. The sheath material may in some embodiments have a strain-hardening exponent between 0.1 and 0.8 and preferably has a strain-hardening exponent between 0.3 and 0.7. Note that in some embodiments a sheath of non-drawn polymer may be extruded over a core polymer which has already been drawn in which case, the sheath elastic constant, plasticity and elongation at break values reflect the properties of the material without drawing. However, the sheath material may be annealed or otherwise treated after extrusion in order to increase the crystallinity and strength (and therefore stiffness). As a further aid to selecting appropriate materials from which to form the core and sheath of a suture as disclosed herein, reference may be made to a treatise that provides relevant material properties, for example, Mark, J. E., ed. Physical Properties of Polymers Handbook. American Institute of Physics Press, Woodbury, N.Y., 1996; Manual for the Rubber Industry, 2d ed. Bayer AG, Akron, Ohio, 1993; The Vanderbilt Rubber Handbook, 3d ed. R. T. Vanderbilt Co., Norwalk, Conn., 1990; Polymer Data Handbook 1999 by Oxford University Press, Inc.; Encyclopedia of Polymer Science and Engineering, edited by H. F. Mark, et al. Wiley-Interscience, New York, 1989; Polymer Handbook (4th Edition) edited by: Brandrup, J.; Immergut, Edmund H.; Grulke, Eric A.; Abe, Akihiro; Bloch, Daniel R. 2005 John Wiley & Sons; including more recent editions of each of the foregoing. Suitable test methods for measuring the properties of a material may be found, for example, in testing protocols issued by ASTM International, 100 Barr Harbor Drive, West Conshohocken, Pennsylvania, USA.

It is another advantage of the present invention that the sheath and/or outer layers/jacket of the braided suture may desirably incorporate materials that further promote tissue engagement. In addition to tissue engagement at the retainers, use of tissue engagement-promoting materials in at least part of the suture sheath surface (whether or not such materials also make up all or part of the retainers) can enhance the ability of the sutures to stay in place. One such class of tissue engagement-promoting materials are porous polymers that can be extruded, including both microporous polymers and polymers that can be extruded with bubbles (whether bioabsorbable or nonbioabsorbable). A suture synthesized with such materials in the sheath can have a three-dimensional lattice structure that increases tissue engagement surface area and permits tissue infiltration into the suture body itself, thus having a sheath structure that promotes successful suture use. Moreover, by optimizing pore size, fibroblast ingrowth can be encouraged, further facilitating the suture to be anchored in the tissue. Furthermore, an agent can be utilized in conjunction with the suture (introduced separately or adhered to the suture or incorporated into a material of the suture) to encourage fibrosis. Fibrosis-inducing agents which may be used in conjunction with a self-retaining sutures in accordance with the present invention are described in U.S. Patent 7,166,570 titled "Medical Implants And Fibrosis-Inducing Agents" to Hunter et al.

Additionally, self-retaining sutures described herein may be provided with therapeutic compositions including, for example, compositions to promote healing and prevent undesirable effects such as scar formation, infection, pain, and so forth. This can be accomplished in a variety of manners, including for example: (a) by directly affixing to the suture a formulation (e.g., by either spraying the suture with a polymer/drug film, or by dipping the suture into a polymer/drug solution), (b) by coating the suture with a substance such as a hydrogel which will in turn absorb the composition, (c) by interweaving formulation-coated fiber or thread (or the polymer itself formed into a fibers or threads) into the suture structure in the case of multifilamentary sutures, (d) constructing the suture itself with a composition. Such compositions may include without limitation anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents.

The particular application of the suture may guide the choice of therapeutic agent that is applied to the suture; for example, self-retaining sutures having anti-proliferative coatings may be used in closing tumor excision sites, while self-retaining sutures with fibrosing coatings may be used in tissue repositioning procedures and those having anti-scarring coatings may be used for wound closure on the skin. As well, the structure of the suture may influence the choice and extent of coating; for example, sutures having an expanded segment may include a fibrosis-inducing composition on the expanded segment to further secure the segment in position in the tissue. Coatings may also include a plurality of compositions either together or on different portions of the suture, where the multiple compositions can be selected either for different purposes (such as combinations of analgesics, anti-infective and anti-scarring agents) or for the synergistic effects of the combination.

The particular application of the suture may guide the method of applying the therapeutic agent to the suture. The particular purpose to which the suture is to be put or the composition may determine whether a fully-coated or selectively-coated suture is appropriate; for example, with lubricious coatings, it may be desirable to selectively coat the suture, leaving, for instance, the tissue-engaging surfaces of the sutures uncoated in order to prevent the tissue engagement function of those surfaces from being impaired. On the other hand, coatings such as those comprising such compounds as anti-infective agents may suitably be applied to the entire suture, while coatings such as those comprising fibrosing agents may suitably be applied to all or part of the suture (such as the tissue-engaging surfaces). For example, a composition can be applied to the suture before the retainers are formed, so that when the retainers engage, the engaging surface is substantially free of the coating. In this way, tissue being sutured contacts a coated surface of the suture as the suture is introduced, but when the retainer engages, a non-coated surface of the retainer contacts the tissue. Alternatively, the suture may be coated after or during formation of retainers on the suture if, for example, a fully-coated rather than selectively-coated suture is desired. In yet another alternative, a suture may be selectively coated either during or after formation of retainers by exposing only selected portions of the suture to the coating.

### Clinical Uses Of Self-Retaining Braided Sutures

In addition to the general wound closure and soft tissue repair applications, self-retaining sutures can be used in a variety of other indications.

Self-retaining sutures described herein may be used in various dental procedures, i.e., oral and maxillofacial surgical procedures and thus may be referred to as "self-retaining dental sutures." The above-mentioned procedures include, but are not limited to, oral surgery (e.g., removal of impacted or broken teeth), surgery to provide bone augmentation, surgery to repair dentofacial deformities, repair following trauma (e.g., facial bone fractures and injuries), surgical treatment of odontogenic and non-odontogenic tumors, reconstructive surgeries, repair of cleft lip or cleft palate, congenital craniofacial deformities, and esthetic facial surgery. Self-retaining dental sutures may be degradable or non-degradable, and may typically range in size from USP 2-0 to USP 6-0.

Self-retaining sutures described herein may also be used in tissue repositioning surgical procedures and thus may be referred to as "self-retaining tissue repositioning sutures". Such surgical procedures include, without limitation, face lifts, neck lifts, brow lifts, thigh lifts, and breast lifts. Self-retaining sutures used in tissue repositioning procedures may vary depending on the tissue being repositioned; for example, sutures with larger and further spaced-apart retainers may be suitably employed with relatively soft tissues such as fatty tissues.

Self-retaining sutures described herein may also be used in microsurgical procedures that are performed under a surgical microscope (and thus may be referred to as "self-retaining microsutures"). Such surgical procedures include, but are not limited to, reattachment and repair of peripheral nerves, spinal microsurgery, microsurgery of the hand, various plastic microsurgical procedures (e.g., facial reconstruction), microsurgery of the male or female reproductive systems, and various types of reconstructive microsurgery. Microsurgical reconstruction is used for complex reconstructive surgery problems when other options such as primary closure, healing by secondary intention, skin grafting, local flap transfer, and distant flap transfer are not adequate. Self-retaining microsutures have a very small caliber, often as small as USP 9-0 or USP 10-0, and may have an attached needle of corresponding size. The microsutures may be degradable or non-degradable.

Self-retaining sutures as described herein may be used in similarly small caliber ranges for ophthalmic surgical procedures and thus may be referred to as "ophthalmic self-retaining sutures". Such procedures include but are not limited to keratoplasty, cataract, and vitreous retinal microsurgical procedures. Ophthalmic self-retaining sutures may be degradable or non-degradable, and have an attached needle of correspondingly-small caliber. Self-retaining sutures can be used in a variety of veterinary applications for a wide number of surgical and traumatic purposes in animal health.

The present disclosure includes many embodiments including, but not limited to the embodiments described in the following paragraphs. It should be understood by those skilled in the art that it is not intended to limit the disclosure to the specific embodiments described. Various modifications, omissions, and additions may be made to the disclosed embodiments without materially departing from the novel teachings and advantages of the disclosure, particularly in light of the foregoing teachings. Specific embodiments of the present disclosure include, without limitation: A suture comprising: a braided core; a sheath which covers said core; and retainers formed in said sheath.

A suture comprising: a braided core made of a first material; a sheath which covers said core, said sheath made of a second material; and retainers formed in said sheath; wherein said first material has at least one of greater tensile strength and greater elasticity than said second material and said second material has greater plasticity than said first material.

A suture comprising: a braided core wherein said braided core includes an ultra high molecular weight polyethylene multi-filament core with a braided polyester jacket; a sheath that covers said core wherein said sheath includes a polyester; and retainers formed in said sheath.

The suture of paragraph 128, 129, 130, 132, 133, 134, 135, 136, 137, 138, 139, 140, 141, 142, 143, 144 or 145 wherein said retainers are heat-treated after being formed to increase stiffness.

The suture of paragraph 128, 129, 130, 131, 133, 134, 135, 136, 137, 138, 139, 140, 141, 142, 143, 144 or 145 wherein said braided core is made of a first material and wherein the sheath is made from a second material, and wherein the first material has at least one of greater tensile strength and greater elasticity than said second material.

The suture of paragraph 128, 129, 130, 131, 132, 134, 135, 136, 137, 138, 139, 140, 141, 142, 143, 144 or 145 wherein said braided core is made of a first material and wherein the sheath is made from a second material, and wherein the second material has a larger plasticity than said first material.

The suture of paragraph 128, 129, 130, 131, 132, 133, 135, 136, 137, 138, 139, 140, 141, 142, 143, 144 or 145 wherein said retainers are plastically deformed into an elevated configuration.

The suture of paragraph 128, 129, 130, 131, 132, 133, 134, 137, 138, 139, 140, 141, 142, 143, 144 or 145 wherein said braided core is ribbon shaped.

The suture of paragraph 128, 129, 130, 131, 132, 133, 134, 137, 138, 139, 140, 141, 142, 143, 144 or 145 wherein said braided core is tube shaped.

The suture of paragraph 128, 129, 130, 131, 132, 133, 134, 135, 136, 138, 139, 140, 141, 142, 143, 144 or 145 wherein said retainers are formed in said sheath and partially in said braided core.

The suture of paragraph 128, 129, 130, 131, 132, 133, 134, 135, 136, 137, 140, 141, 142, 143, 144 or 145 wherein said sheath is shrunk over said braided core.

The suture of paragraph 128, 129, 130, 131, 132, 133, 134, 135, 136, 137, 140, 141, 142, 143, 144 or 145 wherein said sheath is extruded over said braided core.

The suture of paragraph 128, 129, 130, 131, 132, 133, 134, 135, 136, 137, 138, 139, 141, 142, 143, 144 or 145 wherein said braided core includes an ultra high molecular weight polyethylene multi-filament core with a braided polyester jacket.

The suture of paragraph 128, 129, 130, 131, 132, 133, 134, 135, 136, 137, 138, 139, 140, 142, 143, 144 or 145 wherein said sheath includes a polyester.

The suture of paragraph 128, 129, 130, 131, 132, 133, 134, 135, 136, 137, 138, 139, 140, 141, 143, 144 or 145 wherein said braided core includes a non-absorbable material.

The suture of paragraph 128, 129, 130, 131, 132, 133, 134, 135, 136, 137, 138, 139, 140, 141, 142, 144 or 145 wherein said sheath is coated onto said braided core by one of dipping or spraying.

The suture of paragraph 128, 129, 130, 131, 132, 133, 134, 135, 136, 137, 138, 139, 140, 141, 142, 143, or 145 wherein said retainers are formed in said core by an angled cut which passes at least partially through said sheath without entering said braided core.

The suture of paragraph 128, 129, 130, 131, 132, 133, 134, 135, 136, 137, 138, 139, 140, 141, 142, 143 or 144 wherein: the core includes an ultra high molecular weight polyethylene; and the sheath includes a polyester.

A method of making a suture comprising the steps of: forming a braided core; forming a sheath covering said braided core; and forming a plurality of retainers at least in the sheath.

The method of paragraph 146 comprising: forming a braided core from a first material; forming a sheath from a second material covering said braided core; and wherein said first material has at least one of greater tensile strength and greater elasticity than said second material and said second material has greater plasticity than said first material.

A method of making a suture comprising the steps of: forming a braided core; forming a sheath covering said braided core; and forming a plurality of retainers at least in the sheath; wherein said braided core forming step includes forming an ultra high molecular weight polyethylene multi-filament core with a braided polyester jacket; and wherein said sheath forming step includes coating said braided core with polyester; and wherein said braided core forming step includes selecting said braided core to have at least one of greater tensile strength, greater elasticity, or greater flexibility than said sheath; and wherein said sheath forming step includes, selecting said sheath to have a larger plastic deformation zone than said braided core.

A method of making a suture comprising the steps of: forming a braided core; forming a sheath over said core; forming retainers in said sheath; and wherein the braided core forming step includes selecting said braided core to have at least one of greater tensile strength, greater elasticity, or greater flexibility than said sheath; and wherein said sheath forming step includes selecting said sheath to have a larger plastic deformation zone than said braided core.

The method of paragraph 146, 147, 148, 149, 151, 152, 153, 154, 155, 156, 157, 158, 159, 160, 161, 162, 163, 164, 165, 166, 167, 168 or 169 including the step of plastically deforming at least some of the plurality of retainers into an elevated configuration.

The method of paragraph 146, 147, 148, 149, 150, 152, 153, 154, 155, 156, 157, 158, 159, 160, 161, 162, 163, 164, 165, 166, 167, 168 or 169 including the step of: elevating at least some of the plurality of retainers after forming said retainers.

The method of paragraph 146, 147, 148, 149, 150, 151, 153, 154, 155, 156, 157, 158, 159, 160, 161, 162, 163, 164, 165, 166, 167, 168 or 169 including the step of: permanently deforming at least some of the plurality of retainers.

The method of paragraph 146, 147, 148, 149, 150, 151, 152, 154, 155, 156, 157, 158, 159, 160, 161, 162, 163, 164, 165, 166, 167, 168 or 169 including selecting a material of said braided core to have at least one of greater tensile strength, greater elasticity, or greater flexibility than a material of said sheath.

The method of paragraph 146, 147, 148, 149, 150, 151, 152, 153, 155, 156, 157, 158, 159, 160, 161, 162, 163, 164, 165, 166, 167, 168 or 169 including selecting a sheath material to have a larger plastic deformation zone than a braided core material.

The method of paragraph 146, 147, 148, 149, 150, 151, 152, 153, 154, 157, 158, 159, 160, 161, 162, 163, 164, 165, 166, 167, 168 or 169 including forming the braided core as a ribbon or sheet.

The method of paragraph 146, 147, 148, 149, 150, 151, 152, 153, 154, 157, 158, 159, 160, 161, 162, 163, 165, 164, 166, 167, 168 or 169 including forming said braided core as a tube or ring.

The method of paragraph 146, 147, 148, 149, 150, 151, 152, 153, 154, 155, 156, 158, 159, 160, 161, 162, 163, 164, 165, 166, 167, 168 or 169 including forming the retainers in the sheath and partially in said braided core.

The method of paragraph 146, 147, 148, 149, 150, 151, 152, 153, 154, 155, 156, 157, 159, 160, 161, 162, 163, 164, 165, 166, 167, 168 or 169 including forming said retainers in said sheath so as not to have the retainers penetrate into said core.

The method of paragraph 146, 147, 148, 149, 150, 151, 152, 153, 154, 155, 156, 157, 158, 160, 161, 162, 163, 164, 165, 166, 167, 168 or 169 including elevating said retainers from a surface of said sheath.

The method of paragraph 146, 147, 148, 149, 150, 151, 152, 153, 154, 155, 156, 157, 158, 159, 161, 162, 163, 164, 165, 166, 167, 168 or 169 including selecting said sheath so that said sheath shrinks after the sheath covers said braided core.

The method of paragraph 146, 147, 148, 149, 150, 151, 152, 153, 154, 155, 156, 157, 158, 159, 160, 162, 163, 164, 165, 166, 167, 168 or 169 including extruding the sheath over a braided core.

The method of paragraph 146, 147, 148, 149, 150, 151, 152, 153, 154, 155, 156, 157, 158, 159, 160, 161, 163, 164, 165, 166, 167, 168 or 169 including the step of drawing said braided core and sheath before step of forming the plurality of retainers.

The method of paragraph 146, 147, 148, 149, 150, 151, 152, 153, 154, 155, 156, 157, 158, 159, 160, 161, 162, 164, 165, 166, 167, 168 or 169 including forming said retainers using one of a blade cutting technique or a laser cutting technique.

The method of paragraph 146, 147, 148, 149, 150, 151, 152, 153, 154, 155, 156, 157, 158, 159, 160, 161, 162, 163, 165, 166, 167, 168 or 169 including forming said braided core having an ultra high molecular weight polyethylene multi-filament core with a braided polyester jacket.

The method of paragraph 146, 147, 148, 149, 150, 151, 152, 153, 154, 155, 156, 157, 158, 159, 160, 162, 163, 164, 167, 168 or 169 including forming the sheath by coating said braided core with polyester.

The method of paragraph 146, 147, 148, 149, 150, 151, 152, 153, 154, 155, 156, 157, 158, 159, 160, 161, 162, 163, 164, 167, 168 or 169 including forming said sheath by extruding a polyester sheath over said braided core.

The method of paragraph 146, 147, 148, 149, 150, 151, 152, 153, 154, 155, 156, 157, 158, 159, 160, 161, 162, 163, 165, 165, 166, 168 or 169 including using non-absorbable materials to form a strong braided suture with retainers.

The method of paragraph 146, 147, 148, 149, 150, 151, 152, 153, 154, 155, 156, 157, 158, 159, 160, 161, 162, 163, 165, 165, 166, 167 or 169 including dipping or spraying a material on the barbed core to form a sheath.

The method of paragraph 146, 147, 148, 149, 150, 151, 152, 153, 154, 155, 156, 157, 158, 159, 160, 161, 162, 163, 165, 165, 166, 167 or 168 including the step of heat treating the retainers to maintain elevation of the retainers.

A suture comprising: a plurality of suture threads at least one of which has formed thereon retainers; and said plurality of suture threads braided together so that at least some of said retainers extend from said braided threads.

A suture comprising: a plurality of suture threads; said plurality of suture threads braided together; and a plurality of retainers formed in at least one of said suture threads after said plurality of suture threads are braided together.

The suture of paragraph 170, 171, 173, 174, 175, 176 or 177wherein said retainers are formed by one of a cutting blade or a laser.

The suture of paragraph 170, 171, 172, 175, 176 or 177wherein said braided suture threads form a ribbon or sheet.

The suture of paragraph 170, 171, 172, 175, 176 or 177wherein said braided suture threads form a tube or ring.

The suture of paragraph 170, 171, 172, 173, 174, 176 or 177wherein said braided suture threads include an ultra high molecular weight polyethylene material.

The suture of paragraph 170, 171, 172, 173, 174, 175 or 177wherein said retainers are heat treated.

The suture of paragraph 170, 171, 172, 173, 174, 175 or 176 wherein said retainers are plastically deformed into an elevated configuration.

A method of making a suture comprising the steps of: using a plurality of suture threads, at least on of said suture threads has retainers formed thereon; braiding together said plurality of suture threads so that at least some of said retainers extend from said braided threads.

A method of making a suture comprising the steps of: using a plurality of suture threads; braiding said plurality of suture threads together; and forming a plurality of retainers on at least one of said suture threads after said plurality of suture threads are braided together.

The method of paragraph 178, 179, 181, 182, 183, 184 or 185 including forming said retainers using one of a cutting blade or a laser.

The method of paragraph 178, 179, 180, 183, 184 or 185 including forming said braided suture threads into a ribbon or sheet.

The method of paragraph 178, 179, 180, 183, 184 or 185 including forming said braided suture threads into a tube or ring.

The method of paragraph 178, 179, 180, 181, 182, 184 or 185 including using threads that include an ultra high molecular weight polyethylene material.

The method of paragraph 178, 179, 180, 181, 182, 183 or 185 wherein said retainers are plastically deformed into an elevated configuration.

The method of paragraph 178, 179, 180, 181, 182, 183 or 184 wherein said retainers are annealed.

Although the present disclosure has been shown and described in detail with regard to only a few exemplary embodiments of the disclosure, it should be understood by those skilled in the art that it is not intended to limit the invention to the specific embodiments disclosed. Various modifications, omissions, and additions may be made to the disclosed embodiments without materially departing from the novel teachings and advantages of the disclosure, particularly in light of the foregoing teachings. Accordingly, it is intended to cover all such modifications, omissions, additions, and equivalents as may be included within the scope of the invention as defined by the following claims.

## Claims

1. A self-retaining suture (170) comprising:
a ribbon-shaped braided core (173);
a sheath which covers said core (175); and
retainers formed in said sheath (174)
wherein the suture is substantially rectangular in cross-section.

2. The suture of claim 1 wherein said retainers (174) are configured to be heat-treated after being formed to increase stiffness.

3. The suture of any of claims 1-2 wherein said braided core (173) is made of a first material and wherein the sheath (175) is made from a second material, and wherein the first material has at least one of greater tensile strength and greater elasticity than said second material.

4. The suture of any of claims 1-3 wherein said braided core (173) is made of a first material and wherein the sheath (175) is made from a second material, and wherein the second material has a larger plasticity than said first material.

5. The suture of any of claims 1-4 wherein said retainers (174) are configured to be plastically deformed into an elevated configuration.

6. The suture of any of claims 1-5 wherein said retainers (174) are formed in said sheath (175) and partially in said braided core (173).

7. The suture of any of claims 1-6 wherein said sheath (175) is configured to be shrunk over said braided core (173).

8. The suture of any of claims 1-6 wherein said sheath (175) is configured to be extruded over said braided core (173).

9. The suture of any of claims 1-8 wherein said braided core (173) includes an ultra high molecular weight polyethylene multi-filament core with a braided polyester jacket.

10. The suture of any of claims 1-9 wherein said sheath (175) includes a polyester.

11. The suture of any of claims 1-10 wherein said braided core (173) includes a nonabsorbable material.

12. The suture of any of claims 1-11 wherein said retainers (174) are formed in said sheath (175) by an angled cut which passes at least partially through said sheath without entering said braided core (173).

13. The suture of claim 3, wherein:
the first material includes an ultra high molecular weight polyethylene; and the second material includes a polyester.

14. A method of making a self-retaining suture comprising the steps of:
forming a ribbon-shaped braided core (173);
forming a sheath covering said braided core (175); and
forming a plurality of retainers at least in the sheath (174);
wherein the suture is substantially rectangular in cross-section.

15. The method of claim 14 comprising:
forming the braided core (173) from a first material;
forming the sheath (175) from a second material; and
wherein said first material has at least one of greater tensile strength and greater elasticity than said second material.

16. The method of any of claims 14-15 including the step of plastically deforming at least some of the plurality of retainers (174) into an elevated configuration.

17. The method of any of claims 14-16 wherein said sheath (175) is coated onto said braided core (173) by one of dipping or spraying.

## Patentansprüche

1. Selbsthaltende Naht (170), umfassend:
einen bandförmigen geflochtenen Kern (173);
eine Hülle, die den Kern (175) abdeckt; und
in der Hülle (174) ausgebildete Halter,
wobei die Naht einen im Wesentlichen rechteckigen Querschnitt aufweist.

2. Naht nach Anspruch 1, wobei die Halter (174) dazu ausgelegt sind, zur Erhöhung der Steifheit nach der Formung mit Wärme behandelt zu werden.

3. Naht nach einem der Ansprüche 1-2, wobei der geflochtene Kern (173) aus einem ersten Material besteht und wobei die Hülle (175) aus einem zweiten Material besteht, und wobei das erste Material mindestens eines von einer größeren Zugfestigkeit und einer größeren Elastizität als das zweite Material aufweist.

4. Naht nach einem der Ansprüche 1-3, wobei der geflochtene Kern (173) aus einem ersten Material besteht und wobei die Hülle (175) aus einem zweiten Material besteht und wobei das zweite Material eine größere Plastizität aufweist als das erste Material.

5. Naht nach einem der Ansprüche 1-4, wobei die Halter (174) dazu ausgelegt sind, in eine erhöhte Konfiguration plastisch verformt zu werden.

6. Naht nach einem der Ansprüche 1-5, wobei die Halter (174) in der Hülle (175) und teilweise im geflochtenen Kern (173) ausgebildet sind.

7. Naht nach einem der Ansprüche 1-6, wobei die Hülle (175) dazu ausgelegt ist, über den geflochtenen Kern (173) geschrumpft zu werden.

8. Naht nach einem der Ansprüche 1-6, wobei die Hülle (175) dazu ausgelegt ist, über den geflochtenen Kern (173) extrudiert zu werden.

9. Naht nach einem der Ansprüche 1-8, wobei der geflochtene Kern (173) einen multifilen Kern aus ultrahochmolekularem Polyethylen mit einem geflochtenen Polyestermantel aufweist.

10. Naht nach einem der Ansprüche 1-9, wobei die Hülle (175) ein Polyester aufweist.

11. Naht nach einem der Ansprüche 1-10, wobei der geflochtene Kern (173) ein nichtresorbierbares Material aufweist.

12. Naht nach einem der Ansprüche 1-11, wobei die Halter (174) in der Hülle (175) durch einen winkelförmigen Schnitt gebildet werden, der mindestens teilweise durch die Hülle verläuft, ohne in den geflochtenen Kern (173) einzudringen.

13. Naht nach Anspruch 3, wobei:
das erste Material ein ultrahochmolekulares Polyethylen aufweist; und das zweite Material einen Polyester aufweist.

14. Verfahren zur Herstellung einer selbsthaltenden Naht, die folgenden Schritte umfassend:
Bilden eines bandförmigen geflochtenen Kerns (173) ;
Bilden einer Hülle, die den geflochtenen Kern (175) abdeckt; und
Bilden einer Vielzahl von Haltern mindestens in der Hülle (174);
wobei die Naht einen im Wesentlichen rechteckigen Querschnitt aufweist.

15. Verfahren nach Anspruch 14, umfassend:
Bilden des geflochtenen Kerns (173) aus einem ersten Material;
Bilden der Hülle (175) aus einem zweiten Material; und
wobei das erste Material mindestens eines von einer größeren Zugfestigkeit und einer größeren Elastizität als das zweite Material aufweist.

16. Verfahren nach einem der Ansprüche 14-15, umfassend den Schritt der plastischen Verformung mindestens einiger der Vielzahl von Haltern (174) in eine erhöhte Konfiguration.

17. Verfahren nach einem der Ansprüche 14-16, wobei die Hülle (175) auf den geflochtenen Kern (173) durch Tauchen oder Sprühen aufgebracht wird.

## Revendications

1. Fil de suture autorétentif (170) comprenant :
une âme tressée sous forme de ruban (173) ;
une gaine qui recouvre ladite âme (175) ; et
des éléments de retenue formés dans ladite gaine (174),
le fil de suture étant essentiellement rectangulaire en section transversale.

2. Fil de suture selon la revendication 1, dans lequel lesdits éléments de retenue (174) sont configurés pour être traités à la chaleur après avoir été formés de façon à augmenter leur raideur.

3. Fil de suture selon l'une quelconque des revendications 1 et 2, dans lequel ladite âme tressée (173) est constituée d'un premier matériau et dans lequel la gaine (175) est constituée d'un second matériau, et dans lequel le premier matériau présente une résistance à la traction et/ou une élasticité supérieures à celles dudit second matériau.

4. Fil de suture selon l'une quelconque des revendications 1 à 3, dans lequel ladite âme tressée (173) est constituée d'un premier matériau et dans lequel la gaine (175) est constituée d'un second matériau, et dans lequel le second matériau présente une plasticité supérieure à celle dudit premier matériau.

5. Fil de suture selon l'une quelconque des revendications 1 à 4, dans lequel lesdits éléments de retenue (174) sont configurés pour être déformés plastiquement de façon à adopter une configuration élevée.

6. Fil de suture selon l'une quelconque des revendications 1 à 5, dans lequel lesdits éléments de retenue (174) sont formés dans ladite gaine (175) et partiellement dans ladite âme tressée (173) .

7. Fil de suture selon l'une quelconque des revendications 1 à 6, dans lequel ladite gaine (175) est configurée pour être contractée sur ladite âme tressée (173).

8. Fil de suture selon l'une quelconque des revendications 1 à 6, dans lequel ladite gaine (175) est configurée pour être extrudée sur ladite âme tressée (173).

9. Fil de suture selon l'une quelconque des revendications 1 à 8, dans lequel ladite âme tressée (173) comprend une âme multifilament en polyéthylène à très haut poids moléculaire avec une enveloppe en polyester tressée.

10. Fil de suture selon l'une quelconque des revendications 1 à 9, dans lequel ladite gaine (175) comprend un polyester.

11. Fil de suture selon l'une quelconque des revendications 1 à 10, dans lequel ladite âme tressée (173) comprend un matériau non absorbable.

12. Fil de suture selon l'une quelconque des revendications 1 à 11, dans lequel lesdits éléments de retenue (174) sont formés dans ladite gaine (175) par une coupe oblique qui passe au moins partiellement à travers ladite gaine sans pénétrer dans ladite âme tressée (173).

13. Fil de suture selon la revendication 3, dans lequel :
le premier matériau comprend un polyéthylène à très haut poids moléculaire ; et le second matériau comprend un polyester.

14. Procédé de fabrication d'un fil de suture autorétentif comprenant les étapes suivantes :
former une âme tressée sous forme de ruban (173) ;
former une gaine recouvrant ladite âme tressée (175) ; et
former une pluralité d'éléments de retenue au moins dans la gaine (174) ;
dans lequel le fil de suture est essentiellement rectangulaire en section transversale.

15. Procédé selon la revendication 14, comprenant :
former l'âme tressée (173) à partir d'un premier matériau ;
former la gaine (175) à partir d'un second matériau ; et
dans lequel ledit premier matériau présente une résistance à la traction et/ou une élasticité supérieures à celles dudit second matériau.

16. Procédé selon l'une quelconque des revendications 14 et 15, comprenant l'étape consistant à déformer plastiquement au moins certains de la pluralité de d'éléments de retenue (174) de façon à ce qu'ils adoptent une configuration élevée.

17. Procédé selon l'une quelconque des revendications 14 à 16, dans lequel ladite gaine (175) est appliquée sur ladite âme tressée (173) par trempage ou pulvérisation.
